# EUROPÄISCHE PATENTSCHRIFT

(11) **EP 3 593 846 B1**
(45) Veröffentlichungstag und Bekanntmachung des Hinweises auf die Patenterteilung: **29.12.2021**
(21) Anmeldenummer: 19401025.2
(22) Anmeldetag: 01.07.2019
(51) Int. Cl.: A61M 16/00, A61M 16/06, A61M 16/08

(54) **ATEMMASKE UND VERFAHREN ZUR HERSTELLUNG EINER ATEMMASKE**
BREATHING MASK AND METHOD FOR MANUFACTURING SAME
MASQUE RESPIRATOIRE ET PROCÉDÉ DE FABRICATION D'UN MASQUE RESPIRATOIRE

(30) Priorität: 12.07.2018 DE 102018005517
(43) Veröffentlichungstag der Anmeldung: 15.01.2020
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- EP-A1- 2 027 880
- EP-A1- 2 979 719
- WO-A1-2010/139014
- WO-A1-2011/003130
- WO-A1-2013/006899
- WO-A1-2014/129913
- WO-A1-2017/049358
- WO-A1-2018/053589
- DE-A1-102005 041 717
- US-A1- 2008 072 910
- US-A1- 2018 185 597

## Beschreibung

Die vorliegende Erfindung betrifft eine Atemmaske und ein Verfahren zur Herstellung einer solchen Atemmaske. Die Atemmaske umfasst wenigstens eine Maskeneinheit und wenigstens eine Ausströmeinrichtung zum Abführen von Ausatemgas aus der Atemmaske. Die Ausströmeinrichtung weist eine Mehrzahl von Strömungskanälen auf, welche in wenigstens einem Teil der Maskeneinheit angeordnet sind.

Bei Atemmasken für sog. Einschlauchsysteme wird die ausgeatmete Luft in der Regel direkt aus der Atemmaske ins Freie bzw. in die Umgebung abgeführt. Das Ausströmen des Ausatemgases bzw. der Ausatemluft aus der Atemmaske führt allerdings häufig zu unangenehmen Geräuschen. Wenn die Luft auf das Gesicht des Patienten oder seines Bettnachbars trifft, wird auch dies häufig als sehr unangenehm empfunden. Geräusche und Luftströmungen sind besonders störend, wenn die Atemmaske zur Schlaftherapie eingesetzt wird.

Die DE102005041717A1 offenbart eine Atemmaske mit einer Anschlusseinheit für einen Atemgasschlauch und benachbart zu der Anschlusseinheit in der Atemmaske eine Ausströmeinrichtung zum Abführen von Ausatemgas. Die Ausströmeinrichtung weist eine Mehrzahl von Strömungskanälen auf, die in einem Ringspalt in dem Maskenkörper angeordnet und von einem Abdeckring teilweise überdeckt sind, sodass sich ein Ausatemspalt bildet. Nachteilig ist hierbei insbesondere der zweiteilige Aufbau.

Die EP2027880B1 offenbart eine Atemmaske mit einer Anschlusseinheit für einen Atemgasschlauch und in der Anschlusseinheit eine Ausströmeinrichtung zum Abführen von Ausatemgas. Die Ausströmeinrichtung weist eine Mehrzahl von Strömungskanälen auf, die zumindest in einem Ausführungsbeispiel teilweise überdeckt sind. Nachteilig ist hierbei die Anordnung in der Anschlusseinheit.

WO2013/006899 offenbart ebenfalls eine Atemmaske mit einer Anschlusseinheit und einer Ausströmeinrichtung zum Abführen von Ausatemgas.

Es ist daher die Aufgabe der vorliegenden Erfindung, unerwünschte Geräusche und Luftströmungen beim Abführen von Ausatemgas aus einer Atemmaske zu reduzieren bzw. zu vermeiden. Dabei soll die Atemmaske vorzugsweise auch kostenoptimiert herstellbar sein, um möglichst vielen Anwendern bzw. Patienten zur Verfügung zu stehen.

Diese Aufgabe wird mit einer Atemmaske oder einem Verfahren gemäß den unabhängigen Ansprüchen gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die erfindungsgemäße Atemmaske ist insbesondere für ein Beatmungsgerät und/oder für ein Schlaftherapiegerät vorgesehen. Die Atemmaske umfasst wenigstens eine Maskeneinheit und wenigstens eine Ausströmeinrichtung zum Abführen von Ausatemgas aus der Atemmaske. Die Maskeneinheit umfasst wenigstens einen Maskenkörper und wenigstens eine mit dem Maskenkörper verbindbare Anschlusseinheit zum Anschließen einer Atemgasleitung.

Die erfindungsgemäße Atemmaske umfasst eine Maskeneinheit mit wenigstens einer Ausströmeinrichtung zum Abführen von Ausatemgas aus dem Inneren der Atemmaske, wobei die Maskeneinheit wenigstens einen Maskenkörper und wenigstens eine mit dem Maskenkörper verbundenen Maskenwulst und eine mit dem Maskenkörper verbundene Anschlusseinheit zum Anschließen einer Atemgaszuleitung umfasst und wobei die Ausströmeinrichtung eine Mehrzahl von separaten Strömungskanälen aufweist wobei die Ausströmeinrichtung mehrere separate Strömungskanäle aufweist welche jeweils aus zumindest zwei Teil-Strömungskanälen bestehen, wobei die Strömungskanäle voneinander beabstandet und zumindest abschnittsweise kreisförmig oder halbkreisförmig angeordnet sind. Erfindungsgemäß ist die Ausströmeinrichtung als separates Bauteil gefertigt ist, welches in den Maskenkörper integriert ist, wobei die Ausströmeinrichtung durch wenigstens ein Gussteil bereitgestellt wird, wobei das Gussteil durch Einpressen mit dem Maskenkörper verbunden ist, wobei die Ausströmeinrichtung an einer Schnittstelle zwischen dem Maskenkörper und der Anschlusseinheit angeordnet ist.

Die Ausströmeinrichtung umfasst eine Mehrzahl von Strömungskanälen, welche in wenigstens einem Teil der Maskeneinheit angeordnet sind. Dabei umfasst die Ausströmeinrichtung beispielsweise wenigstens neun Strömungskanäle.

Insbesondere sind zwei oder vorzugsweise eine Mehrzahl von Strömungskanälen mit Teil-Strömungskanälen vorgesehen. Dadurch können besonders vorteilhafte Strömungseigenschaften erzielt werden. Insbesondere weisen alle oder wenigstens ein überwiegender Teil der Strömungskanäle wenigstens zwei Teil-Strömungskanäle auf. Die Teil-Strömungskanäle, die einen Strömungskanal bilden sind dabei beispielsweise geometrisch unterschiedlich ausgebildet. Die Strömungskanäle weisen daher geometrisch unterschiedliche Teilabschnitte auf.

Wenigstens einer der Strömungskanäle weist vorzugsweise einen veränderlichen Kanalquerschnitt auf. Vorzugsweise weist wenigstens ein Strömungskanal wenigstens zwei unterschiedliche Kanalquerschnitte auf. So können Geräusche und unerwünschte Strömungen besonders vorteilhaft unterdrückt werden. Möglich und bevorzugt ist auch, dass der Kanalquerschnitt gleichbleibend ausgebildet ist.

Es ist bevorzugt, dass wenigstens einer der Strömungskanäle wenigstens abschnittsweise konisch und/oder kegelförmig ausgebildet ist. Dadurch werden besonders vorteilhafte Strömungseigenschaften erreicht. Insbesondere ist wenigstens einer der Strömungskanäle wenigstens abschnittsweise trichterförmig ausgebildet.

Besonders bevorzugt ist in wenigstens einem der Strömungskanäle wenigstens ein Absatz angeordnet. Ein solcher Absatz bietet eine unaufwendige und zugleich wirksame Möglichkeit zur Reduzierung von Geräuschen und Anströmungen. Insbesondere stellt der Absatz wenigstens einen Teil-Strömungskanal bereit oder ist als eine solche ausgebildet. Der Absatz kann wenigstens eine Erhebung und/oder Vertiefung und/oder Stufe oder dergleichen umfassen oder als eine solche ausgebildet sein.

Die Ausströmeinrichtung ist beispielsweise in dem Maskenkörper oder in der Anschlusseinheit angeordnet.

Die Ausströmeinrichtung ist beispielsweise an einer Schnittstelle zwischen dem Maskenkörper und der Anschlusseinheit angeordnet.

Die Ausströmeinrichtung ist beispielsweise einstückig als Teil des Maskenkörpers oder der Anschlusseinheit ausgebildet.

Die Ausströmeinrichtung ist beispielsweise als separates Bauteil gefertigt, welches in den Maskenkörper oder die Anschlusseinheit integriert ist.

Die Ausströmeinrichtung definiert eine Ebene E3 und der Maskenkörper oder die Anschlusseinheit eine Ebene E4.

Die Ebenen E3 und E4 sind voneinander beabstandet oder fallen zusammen.

Wenn die Ebenen E3 und E4 voneinander beabstandet sind und materialmäßig durch einen Absatz miteinander verbunden sind, sind in dem Absatz die Strömungskanäle angeordnet. Die Ebenen E3 und E4 können zueinander geneigt verlaufen oder parallel zueinander verlaufen.

Erfindungsgemäß weist wenigstens einer der Strömungskanäle wenigstens einen Engstellenabschnitt auf. Insbesondere erstreckt sich der Engstellenabschnitt über wenigstens eine Strecke. Insbesondere ist ein streckenartiger Engstellenabschnitt vorgesehen. Durch einen solchen Engstellenabschnitt können die Strömungseigenschaften und z. B. Strömungsgeschwindigkeit und Druck besonders vorteilhaft beeinflusst werden. Insbesondere stellt der Engstellenabschnitt wenigstens einen Teil-Strömungskanal bereit oder ist als eine solche ausgebildet.

Erfindungsgemäß beträgt die Strecke des Engstellenabschnittes wenigstens ein Achtel der Gesamtlänge des Strömungskanals. Vorzugsweise beträgt die Strecke wenigstens ein Sechstel oder wenigstens ein Fünftel und besonders bevorzugt wenigstens ein Viertel oder wenigstens ein Drittel der Gesamtlänge des Strömungskanals. Möglich ist auch, dass die Strecke wenigstens die Hälfte der Gesamtlänge des Strömungskanals beträgt. Die Strecke kann auch wenigstens zwei Drittel oder drei Viertel oder auch neun Zehntel oder mehr der Gesamtlänge des Strömungskanals betragen. Die Strecke kann auch kürzer ausgebildet sein. Möglich und bevorzugt ist ein punktueller Engstellenabschnitt. Der Engstellenabschnitt kann eine oder zwei oder mehr Engstellen umfassen. Der Strömungskanal kann auch mit zwei oder drei oder mehr Engstellenabschnitten ausgestattet sein.

Erfindungsgemäß weist wenigstens einer der Strömungskanäle in Strömungsrichtung vor dem Engstellenabschnitt wenigstens einen sich trichterartig verengenden Einlassbereich auf. Erfindungsgemäß ist auch, dass wenigstens einer der Strömungskanäle in Strömungsrichtung nach dem Engstellenabschnitt wenigstens einen sich trichterartig aufweitenden Auslassbereich aufweist. Solche Ausgestaltungen bieten eine besonders vorteilhafte Anpassung von Druckverhältnissen und Strömungsgeschwindigkeiten. So können Geräusche erheblich reduziert und unerwünschte Anströmungen weitgehend vermieden werden. Unter einem trichterartigen Bereich wird insbesondere ein sukzessive oder veränderlich zunehmender bzw. abnehmender Kanalquerschnitt verstanden. Der Einlassbereich und/oder der Auslassbereich sind insbesondere trichterförmig bzw. als ein Trichter ausgebildet.

Der Einlassbereich ist dabei im Innenbereich der Atemmaske, dem Gesicht des Patienten zugewandt, angeordnet und der Auslassbereich, dem Einlass gegenüberliegend angeordnet, mündest an die Umgebungsluft.

Zumindest ein Strömungskanal weist eine Länge, beginnend mit dem Einlassbereich bis zum Auslassbereich, von 0,5 bis 5 mm auf, bevorzugt 0,8 - 3 mm. Die Länge des Strömungskanals durchspannt dabei bevorzugt die Ausströmeinrichtung beziehungsweise den Maskenkörper, beginnend mit dem Einlassbereich bis zum Auslassbereich.

Zumindest ein Strömungskanal weist (im Querschnitt) einen Durchmesser oder eine Kantenlänge von 0,1 bis 0,9 mm auf, bevorzugt 0,3 - 7 mm, besonders bevorzugt 0,4 - 0,6 mm.

Wenigstens einer der Strömungskanäle kann wenigstens abschnittsweise einen Kanalquerschnitt mit einem viereckigen Querschnittsprofil aufweisen. Insbesondere weist das viereckige Querschnittsprofil abgerundete Ecken auf. Vorzugsweise ist das viereckige Querschnittsprofil rechteckig ausgebildet. Ein solches Querschnittsprofil ist sowohl in Hinblick auf die Herstellung als auch auf das Strömungsverhalten besonders vorteilhaft. Möglich ist auch, dass das viereckige Querschnittsprofil quadratisch ausgebildet ist. Es kann auch ein dreieckiges oder mehreckiges Querschnittsprofil vorgesehen sein. Möglich ist auch ein rundes oder ovales und beispielsweise elliptisches Querschnittsprofil. Der Kanalquerschnitt kann auch ein Querschnittsprofil mit einer anderen Geometrie aufweisen. Beispielsweise kann ein kreuzförmiges Querschnittsprofil vorgesehen sein.

Vorzugsweise verläuft wenigstens einer der Strömungskanäle schräg in der Maskeneinheit. Bevorzugt ist auch, dass wenigstens zwei der Strömungskanäle schräg zueinander verlaufen. Eine solche Ausgestaltung bietet besonders viele Vorteile. Die schräge Anordnung in der Maskeneinheit bezieht sich insbesondere auf eine Hauptebene der Maskeneinheit. Der Verlauf eines Strömungskanals ist insbesondere durch seine Längsachse bestimmt. Insbesondere verläuft der Strömungskanal in einem Winkel zwischen 10° und 50° und vorzugsweise zwischen 20° und 40° in der Maskeneinheit. Möglich sind auch größere oder kleinere Winkel. Möglich ist auch ein Winkel von 0°. Insbesondere sind die Strömungskanäle in einem Winkel zwischen 20° und 100° und vorzugsweise zwischen 40° und 80° zueinander angeordnet. Möglich sind auch größere oder kleinere Winkel. Möglich ist auch ein Winkel von 0° bzw. eine parallele Anordnung von wenigstens zwei Strömungskanäle. Wenigstens zwei der Strömungskanäle verlaufen insbesondere nicht parallel zueinander.

In allen Ausgestaltungen ist es möglich, dass wenigstens ein Strömungskanal wenigstens abschnittsweise gebogen und/oder kurvenartig und/oder abgewinkelt verläuft. Beispielsweise kann der Strömungskanal um 90° +/- 10° abgewinkelt sein. Möglich sind auch andere Winkel. Es ist ebenfalls möglich, dass wenigstens einer der Strömungskanäle wenigstens abschnittsweise geradlinig verläuft. Beispielsweise ist ein solcher Strömungskanal zylindrisch oder quaderförmig oder mit einer anderen Geometrie ausgestattet. Insbesondere stellt der abgewinkelte Abschnitt wenigstens eine Teil-Strömungskanal bereit oder ist als eine solche ausgebildet.

In einer vorteilhaften Ausgestaltung sind die Strömungskanäle in einem Winkel zwischen 10° und 50° zu einer gemeinsamen Symmetrieachse geneigt, sodass sich eine Abströmrichtung zu einem Randbereich der Maskeneinheit ergibt. Möglich ist auch, dass sich eine Abströmrichtung in eine andere Richtung ergibt. Das bietet eine besonders vorteilhafte Abfuhr der Ausatemluft. Insbesondere sind die Strömungskanäle in einem Winkel zwischen 10° und 50° und vorzugsweise in einem Winkel zwischen 20° und 40° zu der gemeinsamen Symmetrieachse geneigt. Möglich sind auch größere oder kleinere Winkel. Möglich ist auch eine Anordnung der Strömungskanäle in einem Winkel von 0° bzw. ohne Neigung. Die Symmetrieachse ist insbesondere eine Rotationssymmetrieachse.

Die Strömungskanäle sind vorzugsweise ringförmig an der Maskeneinheit angeordnet. Vorzugsweise schneiden sich die Längsachsen der Strömungskanäle in einem gemeinsamen Punkt. Insbesondere sind die Strömungskanäle dabei so angeordnet, dass sich eine schirmförmige Verteilung der Abströmrichtungen ergibt, welche vorzugsweise zu einer Außenseite der Maskeneinheit gerichtet ist. Das bietet viele Vorteile bei der Abfuhr der Ausatemluft. Insbesondere verläuft durch den gemeinsamen Punkt eine gemeinsame Achse und beispielsweise eine Symmetrieachse, zu welcher die Strömungskanäle in einem Winkel und vorzugsweise in dem zuvor beschriebenen Winkel geneigt sind. Die Strömungskanäle können in der ringförmigen Anordnung gleichmäßig oder ungleichmäßig zueinander beabstandet sein.

Vorzugsweise sind die Strömungskanäle ringförmig um die Anschlusseinheit herum in den Maskenkörper angeordnet. Vorzugsweise sind die Strömungskanäle kreisförmig an der Maskeneinheit angeordnet. Insbesondere sind die Strömungskanäle auf einer Kreisbahn angeordnet. Die Strömungskanäle können rotationssymmetrisch angeordnet sein. Die ringförmige Anordnung kann in der Art eines Rechtecks oder Dreiecks oder eines Ovals ausgestaltet sein. Möglich ist auch eine andere Anordnung der Strömungskanäle an der Maskeneinheit.

Es ist bevorzugt, dass die Strömungskanäle über wenigstens einen Teilabschnitt der ringförmigen Anordnung gleichmäßig verteilt sind. Vorzugsweise sind die Strömungskanäle über wenigstens einen anderen Teilabschnitt der ringförmigen Anordnung ausgelassen bzw. verschlossen. So kann ein unerwünschtes Anströmen von Gesichtsbereichen besonders gezielt beeinflusst werden. Insbesondere ist der andere Teilabschnitt bei einer bestimmungsgemäßen Nutzung der Atemmaske zu den Augen gerichtet. Möglich sind auch andere Ausrichtungen. Insbesondere weisen die Strömungskanäle Abströmrichtungen auf, welche von den Augen weg gerichtet sind.

In einer bevorzugten Ausgestaltung weist wenigstens einer der Strömungskanäle eine Auslassöffnung auf, welche quer zu einer Einlassöffnung angeordnet ist. Insbesondere wird dadurch eine Strömung des Ausatemgases vor dem Austreten aus der Atemmaske umgelenkt. Auch diese Ausgestaltung bietet viele Vorteile. Insbesondere ist die Auslassöffnung in einem Winkel zwischen 60° und 120° und vorzugsweise zwischen 80° und 100° zu der Einlassöffnung angeordnet. Beispielsweise ist die Auslassöffnung in einem Winkel von 90° zu der Einlassöffnung angeordnet. In dieser und in anderen Ausgestaltungen kann vorgesehen sein, dass die angegebenen Winkel, insbesondere der Winkel von 90°, um bis zu 5° oder mehr nach oben oder unten abweichen.

In allen Ausgestaltungen ist es besonders bevorzugt, dass die Teil-Strömungskanäle durch wenigstens ein Gießverfahren hergestellt sind. Dadurch können die zuvor beschriebene Teil-Strömungskanäle bzw. die Strömungskanäle besonders wirtschaftlich und zugleich zuverlässig hergestellt werden. Besonders bevorzugt ist die Ausströmeinrichtung durch wenigstens ein Gießverfahren hergestellt. Bevorzugt ist auch, dass die Maskeneinheit und insbesondere der Maskenkörper und/oder die Anschlusseinheit, durch wenigstens ein Gießverfahren hergestellt sind. Die Ausströmeinrichtung und die Maskeneinheit können in einem gemeinsamen Gießverfahren hergestellt sein. Möglich sind auch getrennte Gießverfahren. Vorzugsweise ist ein Spritzgießverfahren vorgesehen. Insbesondere werden die und/oder andere Teile der Atemmaske durch Spritzgießen hergestellt. Möglich sind auch andere Gießverfahren.

Besonders bevorzugt sind die im Gießverfahren hergestellten Teile aus Kunststoff und vorzugsweise aus wenigstens einem Thermoplast. Es können auch wenigstens ein Duroplast und/oder wenigstens ein Elastomer oder dergleichen vorgesehen sein. Möglich sind auch andere Kunststoffe oder Werkstoffe.

Insbesondere ist die Teil-Strömungskanal durch Gießen in wenigstens zwei sich ergänzende Gießwerkzeuge mit jeweils wenigstens einem während des Gießens wenigstens teilweise in wenigstens einen gemeinsamen Strömungskanal hineinragenden Vorsprung hergestellt. Die Teil-Strömungskanal kann durch Gießen in wenigstens ein Gießwerkzeug mit wenigstens einem einziehbaren und während des Gießens wenigstens teilweise in wenigstens einen Strömungskanal hineinragenden Vorsprung hergestellt sein. Eine derart hergestellte Teil-Strömungskanal kann kostengünstig bereitgestellt werden und ermöglicht besonders vorteilhafte Ausgestaltungen der Ausströmeinrichtung. Die Vorsprünge der sich ergänzenden Gießwerkzeuge ragen insbesondere in denselben Strömungskanal hinein. Insbesondere ergänzen sich die wenigsten zwei Vorsprünge zu einer Geometrie des Strömungskanals. Der Vorsprung kann beispielsweise kegelförmig oder stabförmig ausgebildet sein. Möglich sind auch andere Geometrien.

Es ist möglich, dass die Ausströmeinrichtung durch wenigstens ein Gussteil bereitgestellt wird. Vorzugsweise ist das Gussteil durch Umspritzen und/oder Umgießen mit der Maskeneinheit verbunden. Insbesondere ist das Gussteil mit dem Maskenkörper und/oder der Anschlusseinheit in solcher Weise verbunden. Das Gussteil kann durch ein anderes Verfahren mit der Maskeneinheit verbunden sein. Beispielsweise kann das Gussteil mit der Maskeneinheit verschweißt und/oder verklebt sein. Insbesondere ist das Gussteil einstückig mit der Maskeneinheit verbunden. Eine solche schrittweise Herstellung bietet viele Vorteile. Die Ausströmeinrichtung ist insbesondere durch wenigstens ein Gießverfahren hergestellt und durch wenigstens ein weiteres Gießverfahren mit der Maskeneinheit verbunden. Die Maskeneinheit wird insbesondere in dem weiteren Gießverfahren hergestellt. Möglich und bevorzugt ist aber auch, dass die Maskeneinheit, insbesondere der Maskenkörper und/oder die Anschlusseinheit, gemeinsam mit der Ausströmeinrichtung in einem Gießverfahren hergestellt ist.

In allen Ausgestaltungen ist es bevorzugt, dass die Strömungskanäle in dem Maskenkörper und/oder in der Anschlusseinheit angeordnet sind. Das bietet eine besonders vorteilhafte Unterbringung der Strömungskanäle. Es ist möglich, dass alle Strömungskanäle im Maskenkörper oder in der Anschlusseinheit angeordnet sind. Es können auch ein Teil der Strömungskanäle in den Maskenkörper und ein anderer Teil in der Anschlusseinheit angeordnet sein. Insbesondere ist jeweils ein Strömungskanal entweder vollständig in den Maskenkörper oder vollständig in der Anschlusseinheit angeordnet. Insbesondere wird jeweils ein Strömungskanal entweder von dem Maskenkörper oder der Anschlusseinheit begrenzt.

Die Strömungskanäle sind erfindungsgemäß in der Ausströmeinrichtung angeordnet. Die Ausströmeinrichtung kann ein Teil(Bereich) des Maskenkörpers sein und insofern einstückig (mit dem Maskenkörper) sein. Die Ausströmeinrichtung kann ein separates Bauteil sein welches form-, stoff- oder kraftschlüssig mit dem Maskenkörper verbunden ist.

Die Ausströmeinrichtung kann auch alternativ oder ergänzend ein Teil(Bereich) der Anschlusseinheit sein und insofern einstückig (mit der Anschlusseinheit) sein. Die Ausströmeinrichtung kann ein separates Bauteil sein welches form-, stoff- oder kraftschlüssig mit der Anschlusseinheit verbunden ist.

Möglich ist aber auch, dass wenigstens einer der Strömungskanäle teilweise in dem Maskenkörper und teilweise in der Anschlusseinheit angeordnet ist. Dann wird der Strömungskanal insbesondere durch den Maskenkörper und die Anschlusseinheit begrenzt. Die Ausströmeinrichtung kann als separates Teil gefertigt sein und dann den Maskenkörper angepritzt (2K-Prozess)sein.

Die Ausströmeinrichtung kann als separates Teil gefertigt sein welches dann in einen entsprechenden Hinterschnitt im Maskenkörper eingepresst ist. Das hat den Vorteil, dass das filigrane Präzisionsteil in einem optimierten Prozess separat gefertigt ist.

Ziel aller Ausführungsformen ist, eine Strömungsrichtung zu erzielen, die schirmförmig nach außen gerichtet ist, beispielsweise in einem Winkel von 10° - 50° vom Gesicht weg. Daher ist die Austrittsöffnung aller Ausführungsformen in einem Winkel nach außen gerichtet. Die Strömung ist nach vorn vom Gesicht weggelenkt. Daher ist der Austrittswinkel beispielsweise übermäßig radial auszurichten. Denkbar ist auch, den Strömungskanälen verschiedene Ausströmrichtungen zu geben und so die Strömung noch weiter zu streuen. Dadurch wird die Strömungsenergie auf einen größeren Bereich verteilt, was zu geringerer Schallemission führt.

Das erfindungsgemäße Verfahren dient zur Herstellung einer Atemmaske, insbesondere für ein Beatmungsgerät und vorzugsweise für ein Schlaftherapiegerät. Die hergestellte Atemmaske umfasst wenigstens eine Maskeneinheit und wenigstens eine Ausströmeinrichtung zum Abführen von Atemgas aus der Atemmaske. Die Maskeneinheit umfasst wenigstens einen Maskenkörper und wenigstens eine mit dem Maskenkörper verbindbare Anschlusseinheit zum Anschließen einer Atemgaszuleitung. Die Ausströmeinrichtung wird mit einer Mehrzahl von Strömungskanälen ausgestattet. Die Strömungskanäle werden in wenigstens einem Teil der Maskeneinheit und vorzugsweise in dem Maskenkörper und/oder in der Anschlusseinheit angeordnet. Dabei wird die Ausströmeinrichtung mittels wenigstens eines Gießverfahrens, insbesondere mittels Spritzgießen, hergestellt und dabei mit wenigstens einer Teil-Strömungskanal ausgestattet. Das erfindungsgemäße Verfahren bietet viele Vorteile. Beispielsweise ist eine besonders kostengünstige Herstellung der Atemmaske möglich, sodass diese einem großen Kreis von Patienten bzw. Anwendern zur Verfügung gestellt werden kann.

Vorzugsweise dient das Verfahren zur Herstellung der erfindungsgemäßen Atemmaske. Insbesondere ist die zuvor beschriebene Atemmaske nach dem erfindungsgemäßen Verfahren hergestellt. Besonders bevorzugt werden die Strömungskanäle wenigstens teilweise mittels des Gießverfahrens hergestellt und dabei mit wenigstens einer Teil-Strömungskanal ausgestattet.

Vorzugsweise wird die Ausströmeinrichtung in wenigstens zwei sich ergänzende Gießwerkzeuge gegossen. Vorzugsweise ragt dabei während des Gießens von den Gießwerkzeugen jeweils wenigstens ein Vorsprung wenigstens teilweise in wenigstens einen gemeinsamen Strömungskanal hinein. Möglich ist auch, dass die Ausströmeinrichtung in wenigstens ein Gießwerkzeug gegossen wird. Vorzugsweise ragt dabei während des Gießens wenigstens ein Vorsprung wenigstens teilweise in wenigstens einen Strömungskanal hinein. Der Vorsprung wird anschließend eingezogen und dabei wenigstens teilweise aus dem Strömungskanal entfernt.

Die Atemmaske ist insbesondere dazu geeignet und ausgebildet, für ein Schlaftherapiegerät eingesetzt werden. Das Beatmungsgerät ist insbesondere ein Schlaftherapiegerät. Die Anschlusseinheit ist vorzugsweise mit einem Schlauchflansch ausgestattet. Insbesondere ist die Atemmaske für ein Einschlauchsystem vorgesehen. Der Schlauch dient dabei zur Versorgung mit Atemgas. Insbesondere ist zur Abfuhr des Ausatemgases kein Schlauch vorgesehen. Insbesondere ist die Ausströmeinrichtung dazu geeignet und ausgebildet, das Ausatemgas direkt aus der Atemmaske in die Umgebung abzuführen. Bei einer bestimmungsgemäßen Nutzung der Atemmaske sind die Strömungskanäle insbesondere in einem Bereich über dem Mund und/oder über der Nase angeordnet.

Vorzugsweise ist die Anschlusseinheit mit dem Maskenkörper lösbar gekoppelt und insbesondere mittels wenigstens einer Rastverbindung verbunden. Insbesondere ist die Anschlusseinheit in eine Anschlussöffnung des Maskenkörpers einsteckbar. Die Anschlusseinheit kann abgewinkelt oder gerade ausgebildet sein. Die Anschlusseinheit kann wenigstens ein Winkelstück umfassen oder als ein solches ausgebildet sein. Die Maskeneinheit kann wenigstens eine Stirnstütze und/oder wenigstens einen Maskenwulst umfassen. Die Stirnstütze ist insbesondere an dem Maskenkörper befestigt war oder fest mit diesem verbunden. Der Maskenwulst ist insbesondere lösbar mit dem Maskenkörper verbindbar.

Insbesondere ist zumindest ein Teil-Strömungskanal dazu geeignet und ausgebildet, eine Entnahme der Ausströmeinrichtung und/oder wenigstens eines Teils der Maskeneinheit aus einer Gießform formschlüssig zu ermöglichen.

Die zuvor beschriebenen Ausgestaltungen des wenigstens einen Strömungskanals bzw. der wenigstens zwei Strömungskanäle sind vorzugsweise für alle Strömungskanäle oder für einen überwiegenden Teil der Strömungskanäle vorgesehen.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung einer Atemmaske in einer perspektivischen Ansicht;
- Fig. 2: die Atemmaske der Fig. 1 in einer geschnittenen Seitenansicht;
- Fug. 3: eine Atemmaske in einer geschnittenen Seitenansicht mit einer vergrößerten Detailansicht;
- Fig. 4: eine Atemmaske in einer geschnittenen Seitenansicht mit einer vergrößerten Detailansicht;
- Fig. 5: eine Atemmaske in einer perspektivischen Detailansicht;
- Fig. 6: die Atemmaske der Fig. 5 in einer geschnittenen Seitenansicht mit einer vergrößerten Detailansicht;
- Fig. 7: eine Atemmaske in einer perspektivischen Detailansicht;
- Fig. 8: die Atemmaske der Fig. 7 in einer geschnittenen Seitenansicht mit einer vergrößerten Detailansicht;
- Fig. 9: eine Atemmaske in einer perspektivischen Detailansicht;
- Fig. 10: die Atemmaske der Fig. 9 in einer geschnittenen Seitenansicht mit einer vergrößerten Detailansicht;
- Fig. 11: eine Atemmaske in einer perspektivischen Detailansicht;
- Fig. 12: die Atemmaske der Fig. 11 in einer geschnittenen Seitenansicht mit einer vergrößerten Detailansicht;
- Fig. 13: eine Atemmaske in einer perspektivischen Detailansicht;
- Fig. 14: die Atemmaske der Fig. 13 in einer geschnittenen Seitenansicht mit einer vergrößerten Detailansicht;
- Fig. 15: eine Atemmaske in einer perspektivischen Detailansicht;
- Fig. 16: die Atemmaske der Fig. 15 in einer geschnittenen Seitenansicht mit einer vergrößerten Detailansicht;
- Fig. 17: eine Atemmaske in einer perspektivischen Detailansicht;
- Fig. 18: die Atemmaske der Fig. 17 in einer geschnittenen Seitenansicht mit einer vergrößerten Detailansicht;
- Fig. 19: eine Atemmaske in einer perspektivischen Detailansicht;
- Fig. 20: die Atemmaske der Fig. 19 in einer geschnittenen Seitenansicht mit einer vergrößerten Detailansicht;
- Fig. 21: eine Ausgestaltung der Atemmaske der Fig. 19 in einer vergrößerten Detailansicht;
- Fig. 22: eine andere Ausgestaltung der Atemmaske der Fig. 19 in einer vergrößerten Detailansicht;
- Fig. 23: eine Atemmaske in einer perspektivischen Detailansicht;
- Fig. 24: die Atemmaske der Fig. 23 in einer geschnittenen Seitenansicht mit einer vergrößerten Detailansicht;
- Fig. 25: eine Atemmaske in einer perspektivischen Detailansicht;
- Fig. 26: die Atemmaske der Fig. 25 in einer geschnittenen Seitenansicht mit einer vergrößerten Detailansicht;
- Fig. 27: eine rein schematische Darstellung einer Herstellung einer Atemmaske nach dem erfindungsgemäßen Verfahren; und
- Fig. 28: eine rein schematische Darstellung einer Herstellung einer Atemmaske nach dem erfindungsgemäßen Verfahren.
- Fig. 29: eine schematische Darstellung einer Ausströmeinrichtung
- Fig. 30: eine schematische Darstellung einer Atemmaske mit Ausströmeinrichtung am Gesicht eines Patienten.
- Fig. 31 - 34: verschiedene schematische Darstellungen von Atemmasken unterschiedlichen Ausströmeinrichtungen und Strömungskanälen, entsprechend den Fig. 23 - 26

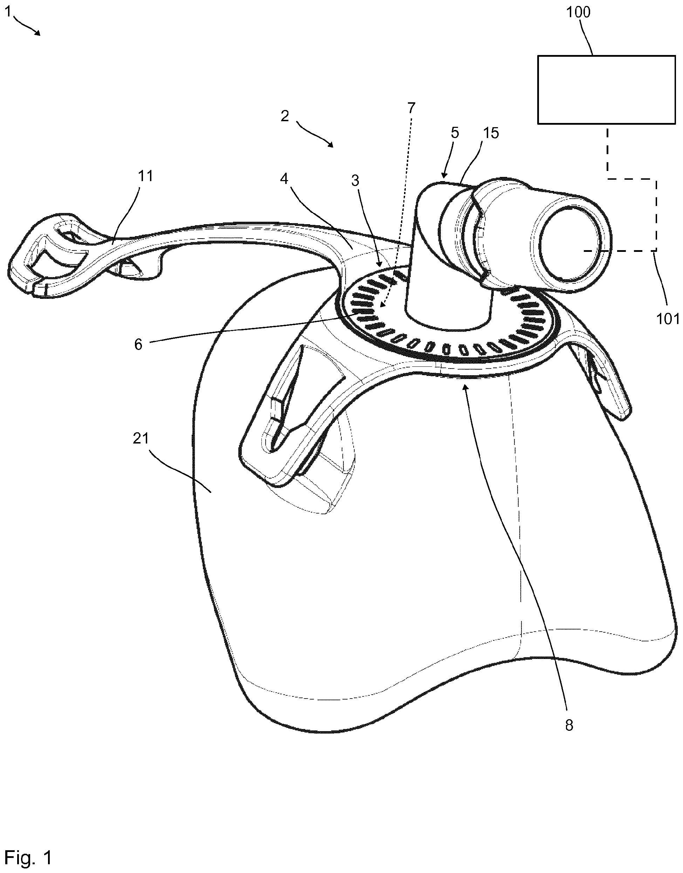

Die Figur 1 zeigt eine erfindungsgemäße Atemmaske 1, welche hier mit einem als Schlaftherapiegerät ausgebildeten Beatmungsgerät 100 verwendet wird. Die Atemmaske 1 umfasst eine Maskeneinheit 2, welche einen Maskenkörper 4 und eine Anschlusseinheit 5 aufweist. An dem Maskenkörper ist hier ein Maskenwulst 21 befestigt. Zudem ist der Maskenkörper 4 mit einer Stirnstütze 11 ausgestattet.

Die Anschlusseinheit 5 ist hier in eine entsprechende Anschlussöffnung des Maskenkörpers 4 eingesteckt und beispielsweise verrastet. Die Anschlusseinheit 5 ist hier mit einem Winkelstück 15 ausgestattet und dient zum Anschließen einer Atemgaszuleitung 101 und beispielsweise eines Atemschlauches an die Maske 1. Der Atemschlauch ist hier an dem Beatmungsgerät 100 angeschlossen.

Um die ausgeatmete Luft aus der Maske 1 abführen zu können, ist eine Ausströmeinrichtung 3 mit einer Mehrzahl von Strömungskanälen 6 vorgesehen. Die ausgeatmete Luft wird über die Strömungskanäle 6 in die Umgebung freigesetzt. Die Ausströmeinrichtung 3 ist hier Teil des Maskenkörpers 4. Dazu verlaufen die Strömungskanäle 6 durch den Maskenkörper 4. Zusätzlich oder alternativ können die Strömungskanäle 6 auch in der Anschlusseinheit 5 vorgesehen sein.

Die Strömungskanäle 6 weisen hier ein rechteckiges Querschnittsprofil mit abgerundeten Ecken auf und sind ringförmig um die Anschlusseinheit 5 herum an dem Maskenkörper 4 angeordnet. Dabei sind die Strömungskanäle 6 gleichmäßig verteilt. In einer hier nicht gezeigten Ausgestaltung kann ein Teilabschnitt der ringförmigen Anordnung mit verschlossenen Strömungskanälen 6 oder sogar ohne Strömungskanäle 6 ausgebildet sein. Ein solcher Teilabschnitt ist beispielsweise zu den Augen gerichtet bzw. unterhalb der Stirnstütze 11 angeordnet.

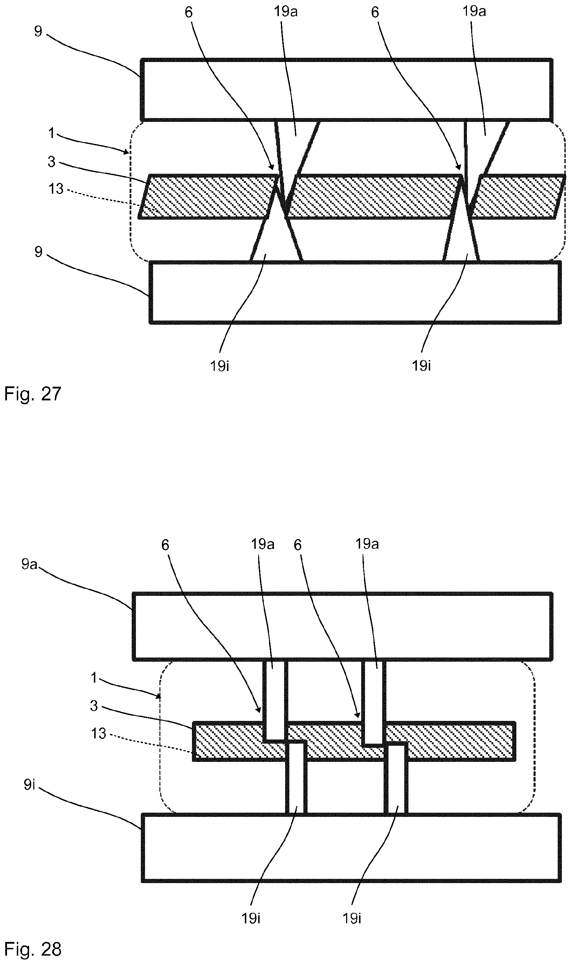

Die Ausströmeinrichtung 5 ist in einem Gießverfahren und beispielsweise durch Spritzgießen hergestellt. Bei der Herstellung wird die Ausströmeinrichtung 3 mit einer in der hier gezeigten Darstellung nicht sichtbaren Teil-Strömungskanal 7 ausgestattet. Um die Ausströmeinrichtung 3 trotz der Teil-Strömungskanal 7 aus einer Gießform entnehmen zu können bzw. um ein formschlüssiges Blockieren der Entnahme zu umgehen, wird das mit Bezug zu den Figuren 27 und 28 näher beschriebene erfindungsgemäße Verfahren eingesetzt.

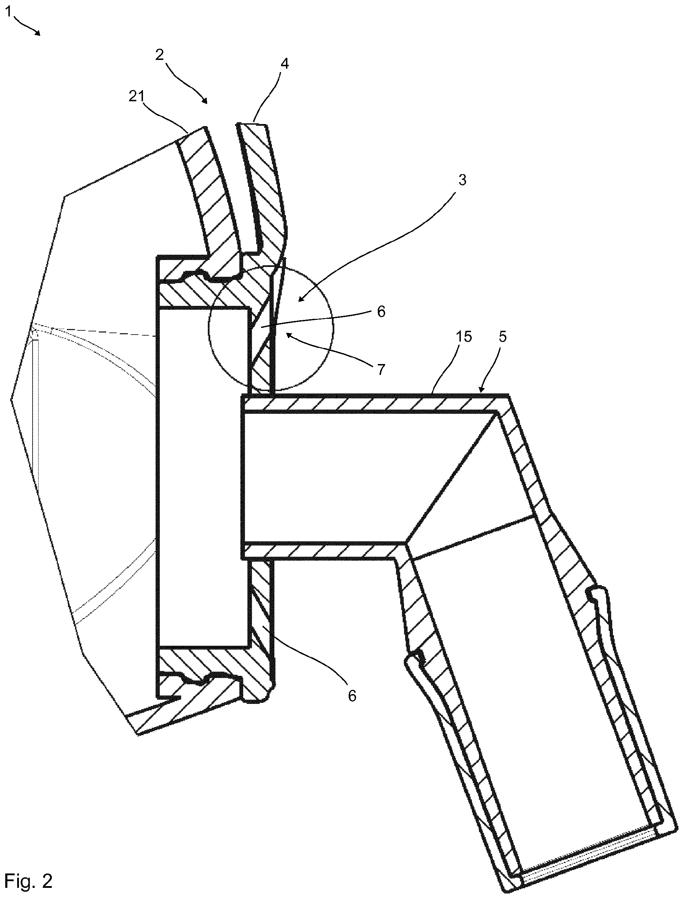

In der Figur 2 ist eine geschnittene Detailansicht der Atemmaske 1 gezeigt, welche den Bereich der Ausströmeinrichtung 3 näher darstellt. Die Ausgestaltung der Strömungskanäle 6 ist dabei besonders gut zu erkennen.

Die Strömungskanäle sind hier mit einem gleichbleibenden bzw. stetigen Kanalquerschnitt ausgestattet. Die Strömungskanäle 6 verlaufen hier schräg in dem Maskenkörper 4 und auch schräg zueinander. Beispielsweise sind die Strömungskanäle 6 hier in einem Winkel zwischen 20° und 40° zu einer gemeinsamen Symmetrieachse geneigt. Die Längsachsen der Strömungskanäle 6 schneiden sich dabei in einem gemeinsamen Punkt. So wird die ausgeatmete Luft zu einem Randbereich der Atemmaske 1 abgeführt.

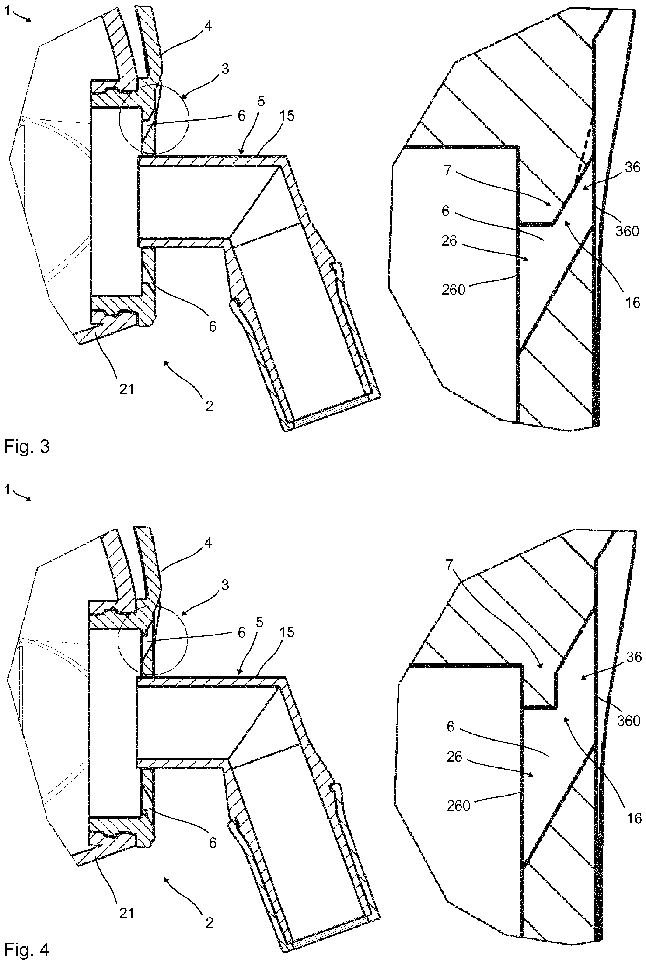

Die Figur 3 zeigt eine Atemmaske 1 mit einer anderen vorteilhaften Ausgestaltung der Ausströmeinrichtung 3. Der kreisförmig umrandete Bereich der Ausströmeinrichtung 3 ist zur besseren Übersichtlichkeit rechts im Bild vergrößert dargestellt.

Die Strömungskanäle 6 sind hier abschnittsweise mit einem veränderlichen Kanalquerschnitt ausgestattet. Im Bereich des veränderlichen Kanalquerschnitts sind die Strömungskanäle 6 beispielsweise konisch oder kegelförmig ausgebildet.

In den Strömungskanälen 6 ist hier jeweils ein Engstellenabschnitt 16 angeordnet. Wie in der vergrößerten Detaildarstellung besonders gut zu erkennen, ist der Engstellenabschnitt 16 hier streckenartig ausgebildet. Der gezeigte Strömungskanal 6 erstreckt sich hier zwischen einer Einlassöffnung 260 und einer Auslassöffnung 360. An die Einlassöffnung 260 schließt sich hier ein sich trichterartig verengender Einlassbereich 26 an, welcher sich bis zu dem Engstellenabschnitt 16 erstreckt. Der Engstellenabschnitt 16 verläuft von dort streckenartig bis zur Auslassöffnung 360.

In einer hier gestrichelt eingezeichneten Ausgestaltung kann der Strömungskanal 6 mit einem sich trichterartig aufweitenden Auslassbereich 36 ausgestattet sein, welcher sich in Strömungsrichtung nach dem Engstellenabschnitt 16 erstreckt. In einer solchen Ausgestaltung tritt die ausgeatmete Luft in den sich trichterartig verengenden Einlassbereich 26 ein und strömt von dort über die Strecke des Engstellenabschnitts 16 weiter bis zu dem sich trichterartig aufweitenden Auslassbereich 36. Dort tritt die Luft über die Auslassöffnung 360 ins Freie.

Die Figur 4 zeigt eine Atemmaske 1 mit einer anderen Ausgestaltung der Ausströmeinrichtung 3. Der kreisförmig umrandete Bereich ist in der rechten Bildhälfte vergrößert dargestellt und zeigt einen exemplarischen Strömungskanal 6.

In dem Strömungskanal 6 ist hier ein Absatz angeordnet, welcher einen Engstellenabschnitt 16 bildet bzw. Teil eines solchen ist. Der Einlassbereich 26 verengt sich hier zunehmend bis zu dem Engstellenabschnitt 16. Nach dem Engstellenabschnitt 16 weitet sich der Strömungskanal 6 wieder auf.

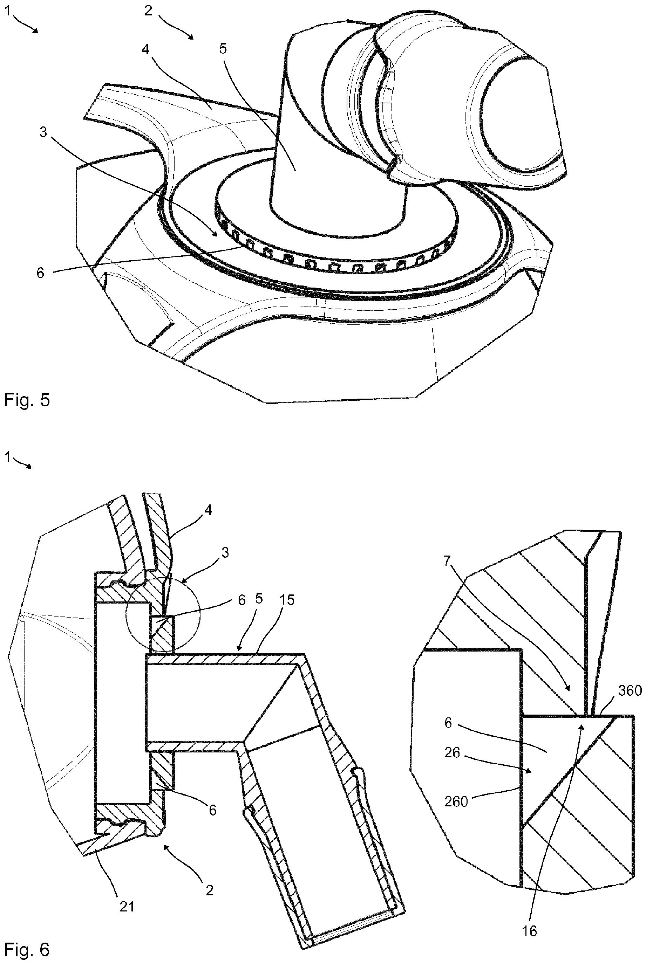

Die Figur 5 zeigt einen Ausschnitt einer beispielhaften Ausgestaltung der Atemmaske 1. Dabei zeigt die Figur 6 eine geschnittene Detaildarstellung der Figur 5, wobei der kreisförmig umrandete Bereich in der rechten Bildhälfte vergrößert dargestellt ist. Die Strömungskanäle weisen hier Auslassöffnungen 360 auf, welche quer zu den jeweiligen Einlassöffnungen 260 angeordnet sind. Die Auslassöffnungen 360 in einem Winkel von 70 - 120°, bevorzug 90° zu den jeweiligen Einlassöffnungen 260 angeordnet. So wird die Strömung der ausgeatmeten Luft vor dem Austreten aus der Atemmaske 1 umgelenkt. Die Einlassöffnung 260 weist einen trichterartigen Bereich auf, der sich zu einer Engstelle 16 verjüngt und dann in die Auslassöffnung 360 übergeht. Zumindest ein Teilbereich der Auslassöffnung 360 befindet sich oberhalb einer Ebene E, die durch den Maskenkörper 4 definiert wird. Der Maskenkörper weist benachbart zu der Auslassöffnung 360 einen Teilbereich auf, der angeschrägt ist und als Strömungsleitfläche dient.

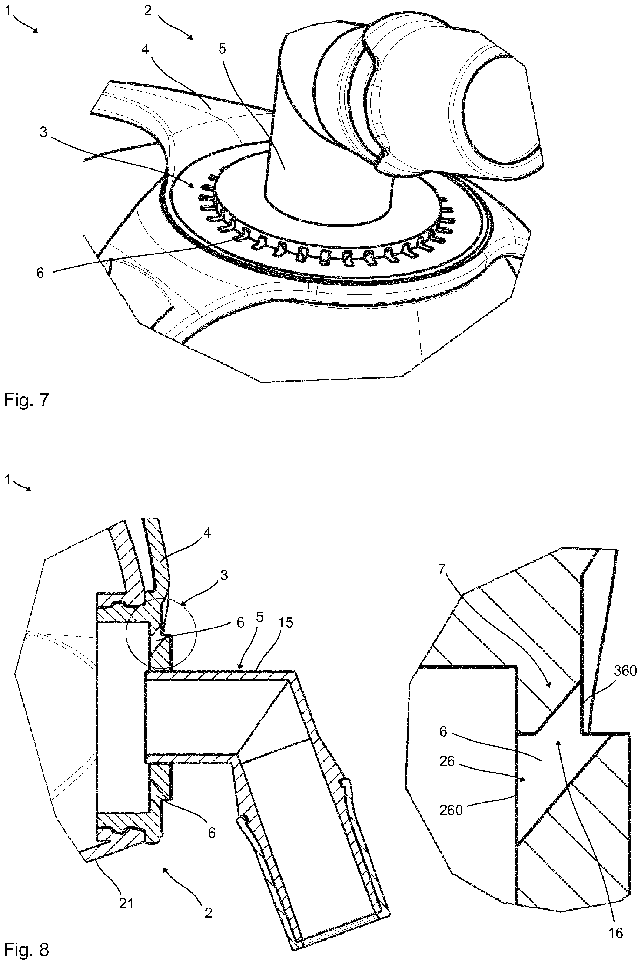

Die Figur 7 zeigt eine Ausgestaltung der erfindungsgemäßen Atemmaske 1. Dabei zeigt die Figur 8 eine geschnittene Detaildarstellung der Figur 7, wobei der kreisförmig umrandete Bereich in der rechten Bildhälfte vergrößert dargestellt ist. Hier ist jeweils nur ein Teil der Auslassöffnung 360 quer zu den jeweiligen Einlassöffnungen 260 angeordnet. Dadurch wird ein besonders vorteilhaftes Strömungsverhalten erreicht.

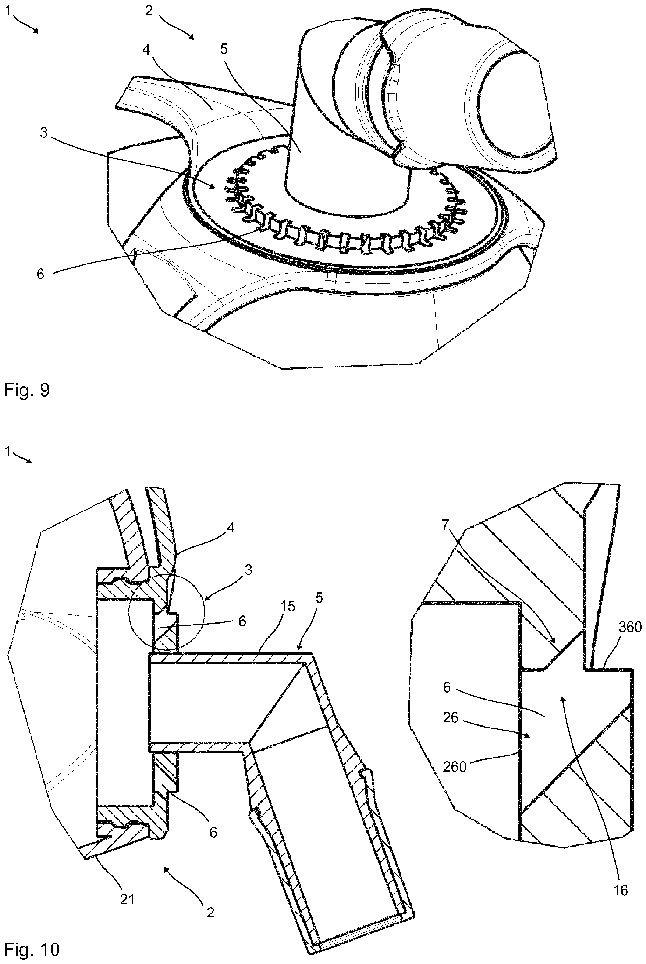

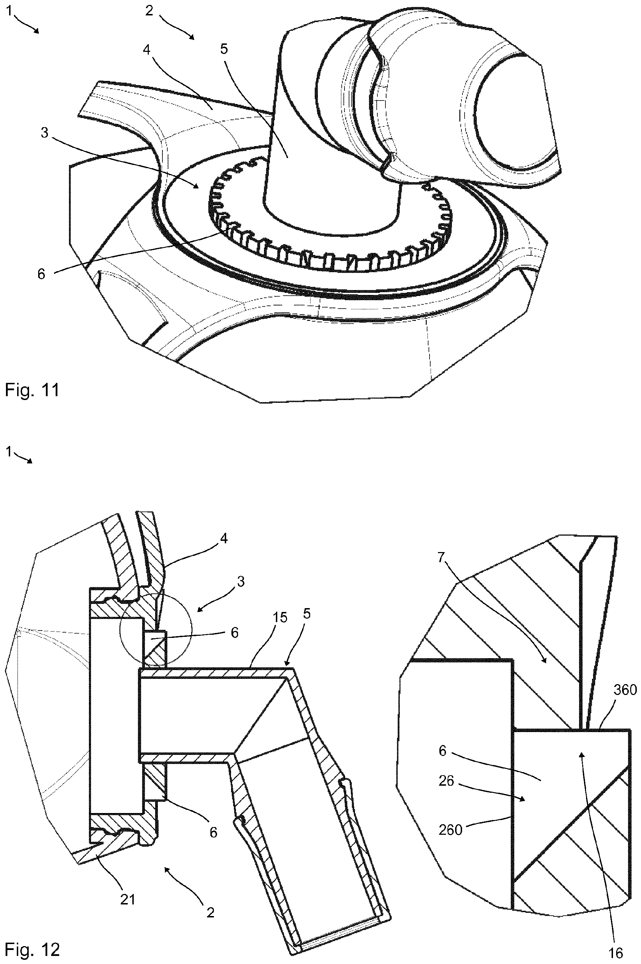

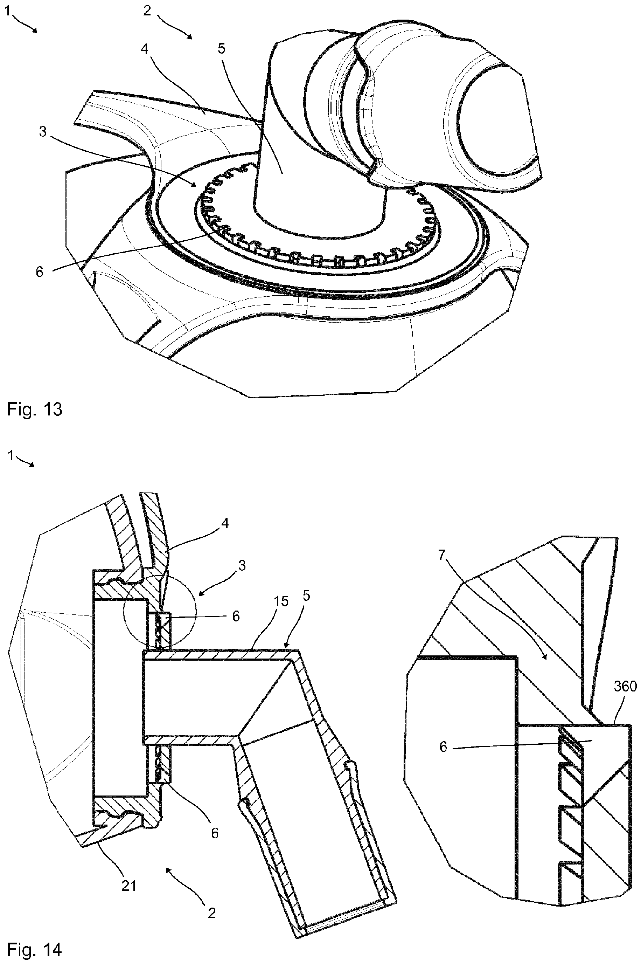

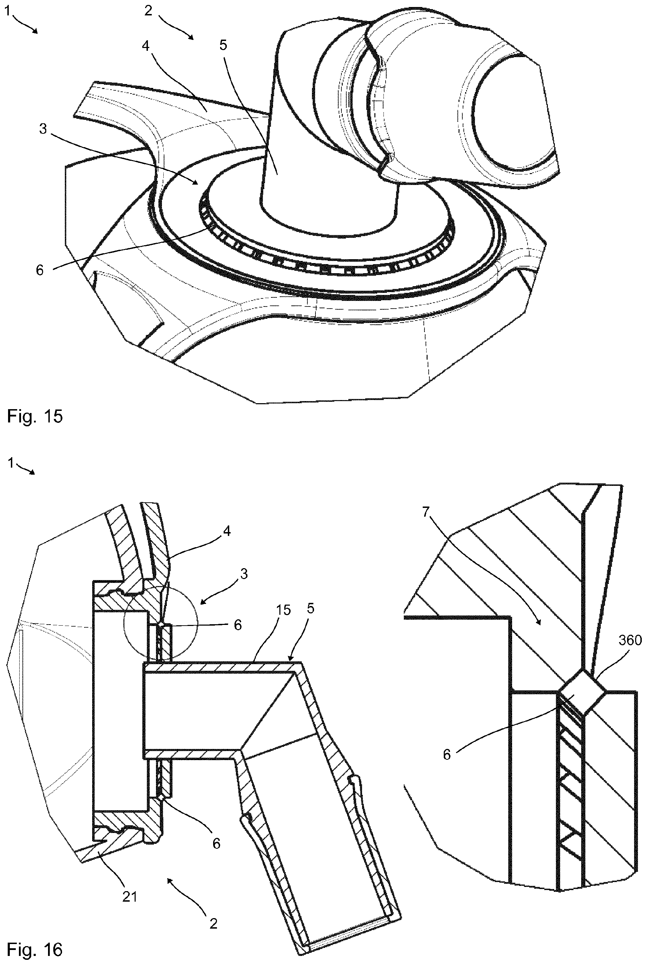

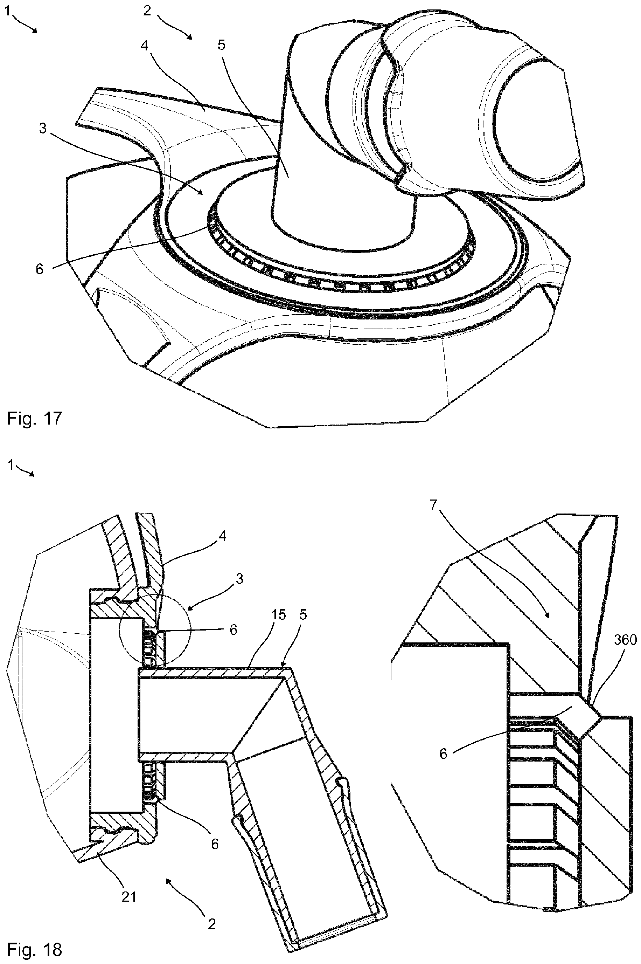

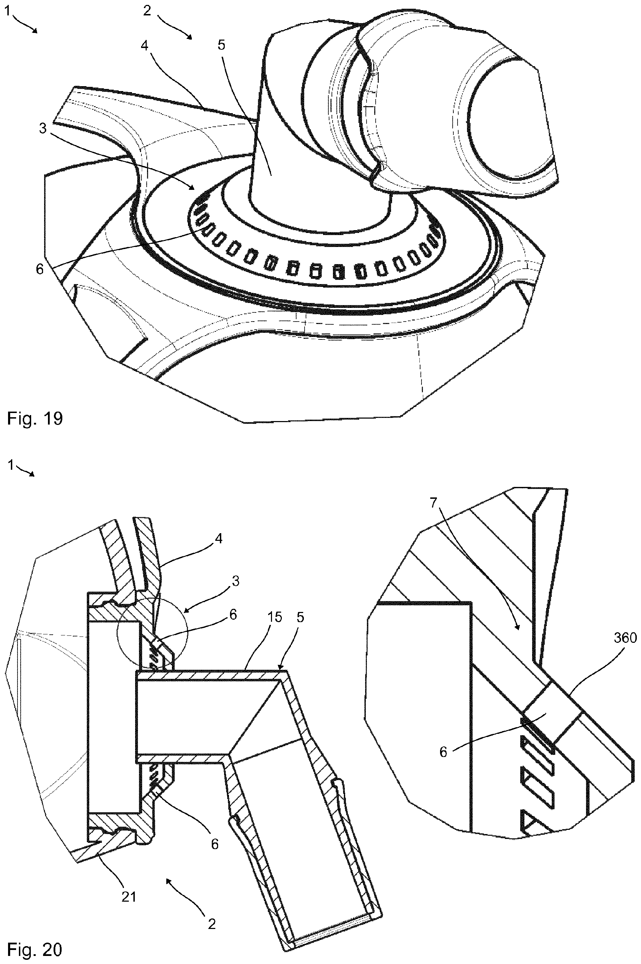

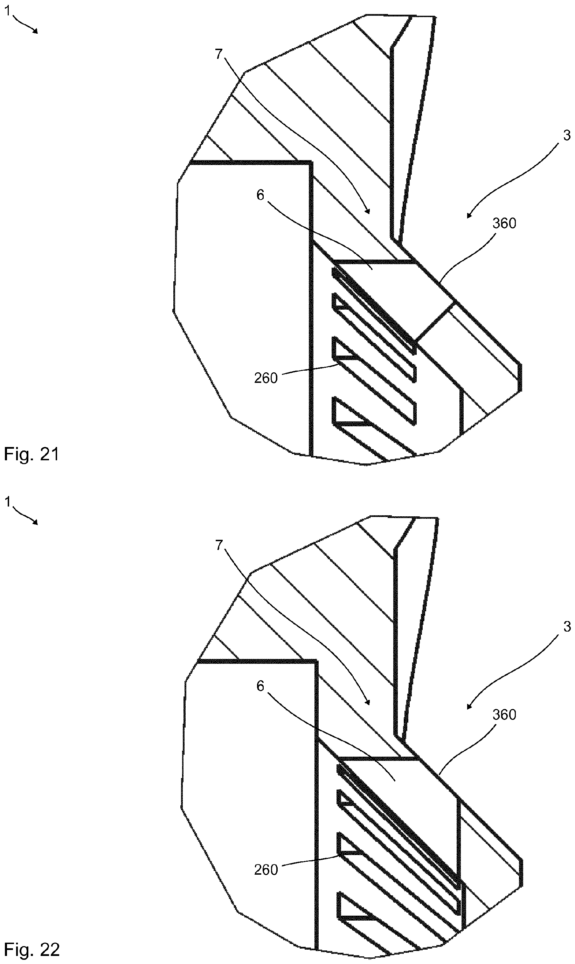

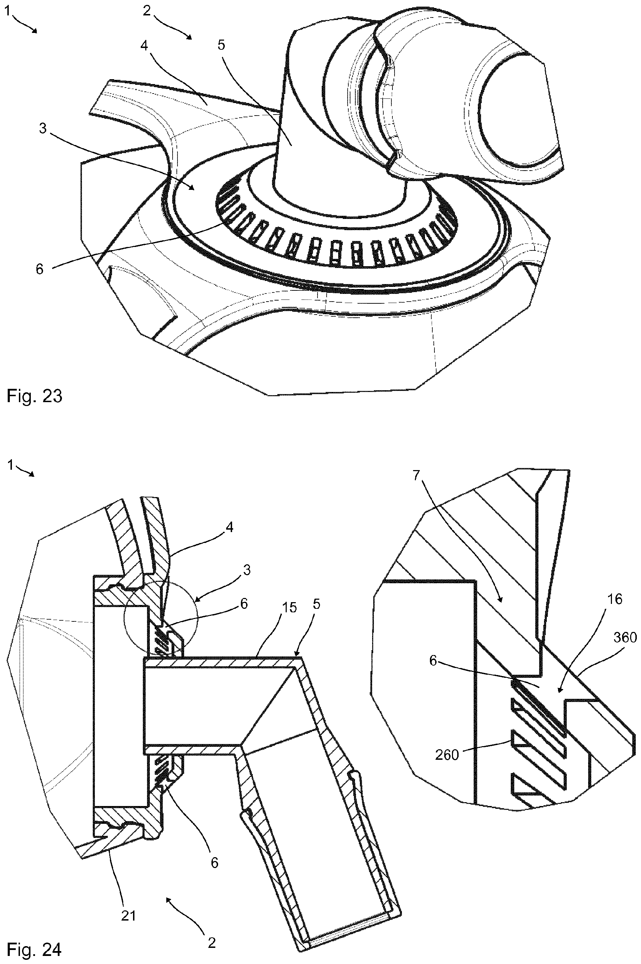

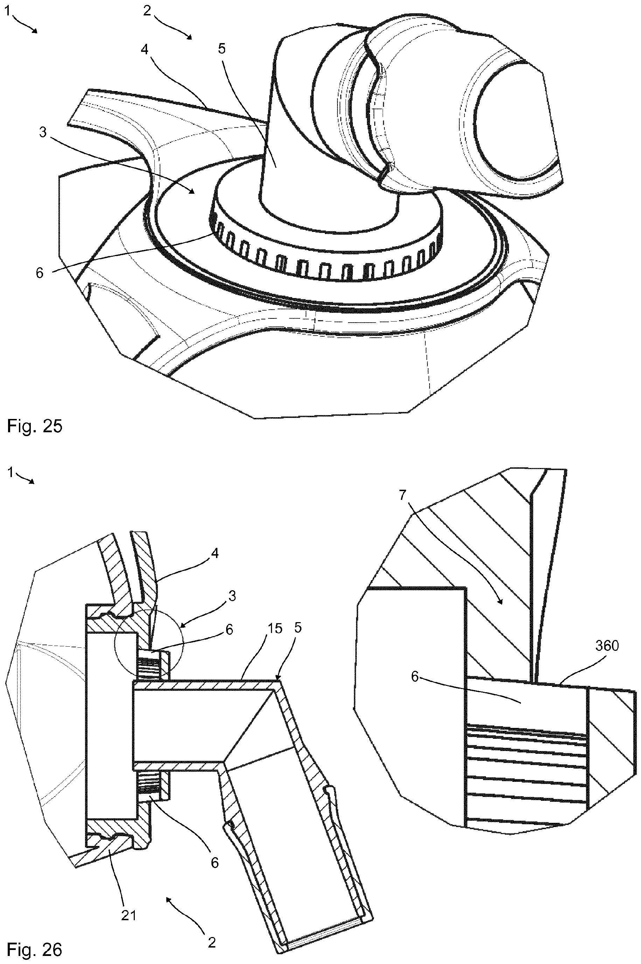

Die Figuren 9, 11, 13, 15, 17 und 19 zeigen jeweils beispielhafte Ausgestaltungen der Atemmaske 1. Dabei zeigen die Figuren 10, 12, 14, 16, 18 und 20 jeweils eine geschnittene Detaildarstellung der jeweils vorhergehenden Figur, wobei der kreisförmig umrandete Bereich in der rechten Bildhälfte vergrößert dargestellt ist. Vorteilhafte Ausgestaltungen der Ausströmeinrichtung 3 der in der Figur 19 gezeigten Atemmaske 1 sind in den Figuren 21 und 22 in vergrößerten Detaildarstellungen gezeigt. Die Figuren 23 und 25 zeigen jeweils eine Ausgestaltung der erfindungsgemäßen Atemmaske 1. Dabei zeigen die Figuren 24 und 26 jeweils eine geschnittene Detaildarstellung der Figuren 23 bzw. 25, wobei der kreisförmig umrandete Bereich in der rechten Bildhälfte vergrößert dargestellt ist.

Die Figur 27 zeigt Gießwerkzeuge 9 für das erfindungsgemäße Verfahren zur Herstellung der Atemmaske 1. Zur Herstellung der Teil-Strömungskanal 7 werden hier zwei sich ergänzende Gießwerkzeuge 9 eingesetzt. Dabei wird die Ausströmeinrichtung 3 beispielsweise im Spritzguss gefertigt. Jedes Gießwerkzeug 9 ist hier mit jeweils zwei Vorsprüngen 19 ausgestattet. Die Vorsprünge sind hier beispielsweise kegelförmig ausgebildet.

Während des Gießens erstreckt sich jeweils ein Vorsprung 19 des einen Gießwerkzeugs 9 mit jeweils einem Vorsprung 19 des anderen Gießwerkzeugs 9 in einen gemeinsamen Strömungskanal 6. Durch die hier gezeigten Gießwerkzeuge 9 und Vorsprünge 19 werden Formtrennungen im Bereich der Strömungskanäle 6 mit einem Verlauf gefertigt, welcher besonders günstige Strömungseigenschaften bietet. Durch die Formtrennungen wird eine Entnahme der Ausströmeinrichtung 3 trotz der Teil-Strömungskanal 7 nicht formschlüssig blockiert.

Dabei kann die Ausströmeinrichtung 3 in einem Gießvorgang zusammen mit der Maskeneinheit 2 und beispielsweise mit dem Maskenkörper 4 oder der Anschlusseinheit 5 gefertigt werden. Möglich ist aber auch, dass die Ausströmeinrichtung 3 als ein zunächst separates Gussteil 13 in einem eigenen Gießverfahren gefertigt wird. Anschließend wird das Gussteil 13 in ein weiteres Gießwerkzeug 9 eingelegt und dort umspritzt. Durch das Umspritzen wird das Gussteil 13 mit der Maskeneinheit 2 und beispielsweise mit dem Maskenkörper 4 oder der Anschlusseinheit 5 verbunden.

Die Figur 28 zeigt die Gießwerkzeuge 9 in einer alternativen Ausgestaltung. Die Vorsprünge 19 sind hier stabförmig ausgebildet.

In einer Ausgestaltung können ein oder mehrere Gießwerkzeuge 9 auch mit einziehbaren Vorsprüngen 19 ausgestattet sein. Dabei ragen die Vorsprünge 19 während des Gießens in einen bzw. mehrere Strömungskanäle 6 hinein. Nach dem Gießen können die Vorsprünge 19 dann eingezogen werden, sodass eine Entnahme der Ausströmeinrichtung 3 trotz der Teil-Strömungskanal 7 nicht formschlüssig blockiert wird.

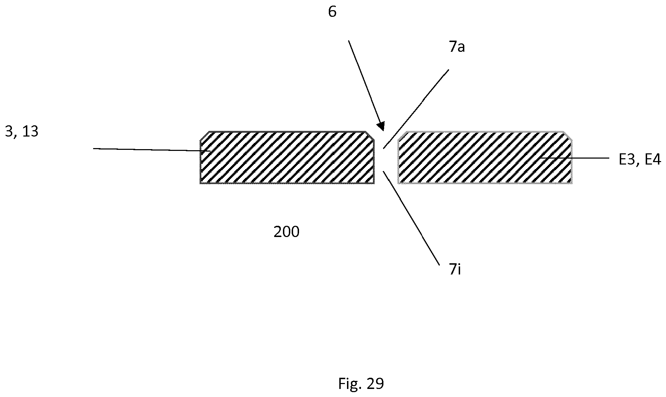

Die Figur 29 zeigt die Ausströmeinrichtung (3) mit zumindest einem Strömungskanal (6) welcher aus zumindest zwei Teil-Strömungskanälen (7a, 7i) besteht. Die Teil-Strömungskanäle (7a, 7i) weisen zumindest abschnittsweise unterschiedliche Geometrien auf, die derart ausgebildet sind, dass sie die strömungstechnischen Anforderungen für den, dem Patienten 200 zugewandten, Innenbereich (7i) und dem, der Umgebungsluft zugewandten, Außenbereich (7a) erfüllen. Die Teil-Strömungskanäle (7a, 7i) können durch einen Gießprozess mit zwei sich ergänzenden Gießwerkzeugen (9) mit unterschiedlich gestalteten Vorsprüngen (19) hergestellt sein.

Die Ausströmeinrichtung (3) kann als Teil des Maskenkörpers (4) oder der Anschlusseinheit (5) ausgebildet sein oder als separates Bauteil gefertigt sein, welches jedoch in den Maskenkörper / die Anschlusseinheit integriert ist. Die Ausströmeinrichtung (3) bildet eine Ebene E3 und der Maskenkörper / die Anschlusseinheit eine Ebene E4 aus. Die Ebenen E3 und E4 stehen beispielsweise senkrecht zueinander. Die Ebenen E3 und E4 können voneinander beabstandet sein oder können zusammenfallen. Die Ebenen E3 und E4 können zueinander geneigt verlaufen oder parallel zueinander. Wenn die Ebenen E3 und E4 voneinander beabstandet sind, sind sie materialmäßig durch einen Absatz oder ein Verbindungsstück (28) miteinander verbunden. In dem Absatz können die Strömungskanäle (6) angeordnet sein. Der Absatz kann beliebige Neigungen aufweisen und die Ebenen E3 und E4, beispielsweise mit einer Krümmung oder einer Geraden, verbinden. Durch die Neigung des Absatzes wird auch die Strömungsrichtung bestimmt.

Figur 29 zeigt, dass die zwei sich ergänzenden Gießwerkzeugen (9) unterschiedlich gestaltete Vorsprünge (19) aufweisen müssen, da diese unterschiedlich gestaltet Teil-Strömungskanäle (7a, 7i) formen, welche zusammen den Strömungskanal (6) bilden.

Die Ausströmeinrichtung 3 der Fig. 29 kann beispielsweise in einer Atemmaske mit wenigstens einer Maskeneinheit (2) verwendet werden, wobei die Maskeneinheit (2) wenigstens einen Maskenkörper (4) und wenigstens eine mit dem Maskenkörper (4) verbindbare Anschlusseinheit (5) zum Anschließen einer Atemgaszuleitung (101) umfasst und wobei die Ausströmeinrichtung mit einer Mehrzahl von Strömungskanälen (6) ausgestattet ist und wobei die Strömungskanäle (6) in der Ausströmeinrichtung in einem Gießprozess mit wenigstens zwei sich ergänzenden Gießwerkzeugen (9) mit jeweils wenigstens einem während des Gießens wenigstens teilweise den Strömungskanal (6) hineinragenden Vorsprüngen (19) hergestellt ist.

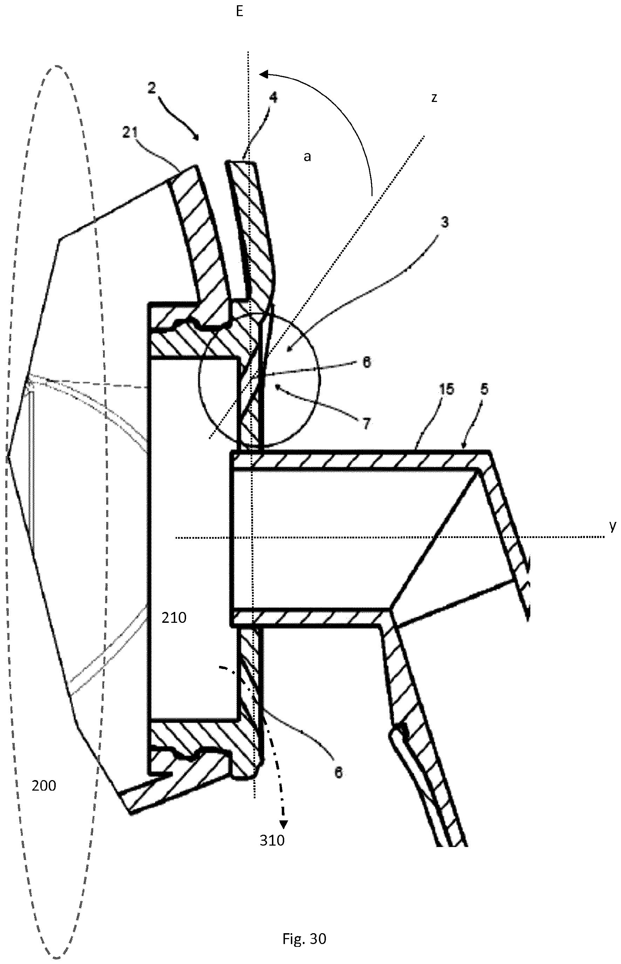

Fig. 30 zeigt eine Atemmaske (1) mit einer Maskeneinheit (2) und mit wenigstens einer Ausströmeinrichtung (3) zum Abführen von Ausatemgas aus dem Inneren der Atemmaske (1), wobei die Maskeneinheit (2) wenigstens einen Maskenkörper (4) und wenigstens eine mit dem Maskenkörper (4) verbundene Anschlusseinheit (5) zum Anschließen einer Atemgaszuleitung (101) umfasst und wobei die Ausströmeinrichtung (3) eine Mehrzahl von separaten Strömungskanälen (6) aufweist, welche in dem Maskenkörper (4) benachbart zu der Anschlusseinheit (5) angeordnet sind, wobei die Ausströmeinrichtung (3) zumindest neun separate Strömungskanäle (6) aufweist welche jeweils aus zumindest zwei Teil-Strömungskanälen (7, 7a, 7i) bestehen, wobei die Strömungskanäle (6) voneinander beabstandet und benachbart zu der oder in der Anschlusseinheit (5) angeordnet sind. Die Teil-Strömungskanäle (7, 7a, 7i) können identisch oder unterschiedlich gestaltet sein.

Die Atemmaske (1) liegt in einem Betriebszustand mit dem Maskenwulst (21) abdichtend an dem Gesicht eines Patienten (200) an und hält dabei einen Überdruck im Inneren der Atemmaske, auf der dem Patienten zugewandten Seite, im Wesentlichen aufrecht, wobei die Ausströmeinrichtung (3) mittels der Strömungskanäle (6) unter Überdruck stehendes Ausatemgas aus dem Inneren (210) der Atemmaske (1) an die Umgebungsluft (310) abführt. Die Atemmaske (1) definiert mit der Anordnung der Ausströmeinrichtung eine Ebene (E) senkrecht zur Achse (y) der Anschlusseinrichtung, wobei die Achse (y) der Anschlusseinrichtung in einem Nutzungszustand am Patienten beispielsweise senkrecht vom Gesicht des Patienten wegführt. Senkrecht bedeutet hier in einem Winkel-Bereich von 70 - 120° relativ zu einer Ebene die das Gesicht aufspannt. Die Strömungskanäle (6) definieren mit ihrer Ausströmrichtung jeweils eine Ebene (z), beispielsweise durch den Bereich ihres kleinsten Querschnitts, wobei die Ebenen (E) und (z) in einem Winkel (a) zwischen 20 und 100°, bevorzugt zwischen 45 und 90° zueinander angeordnet sind.

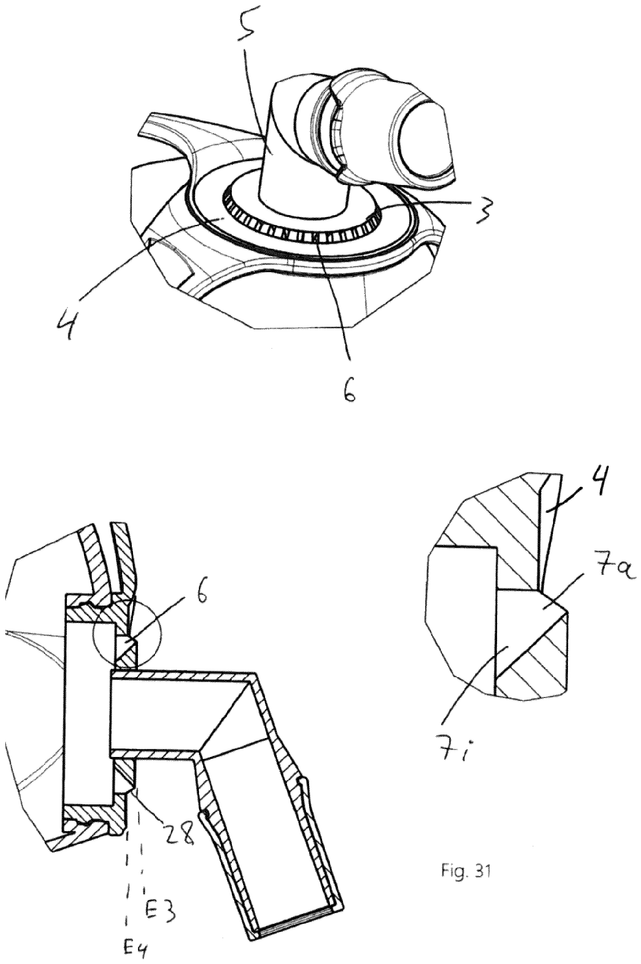

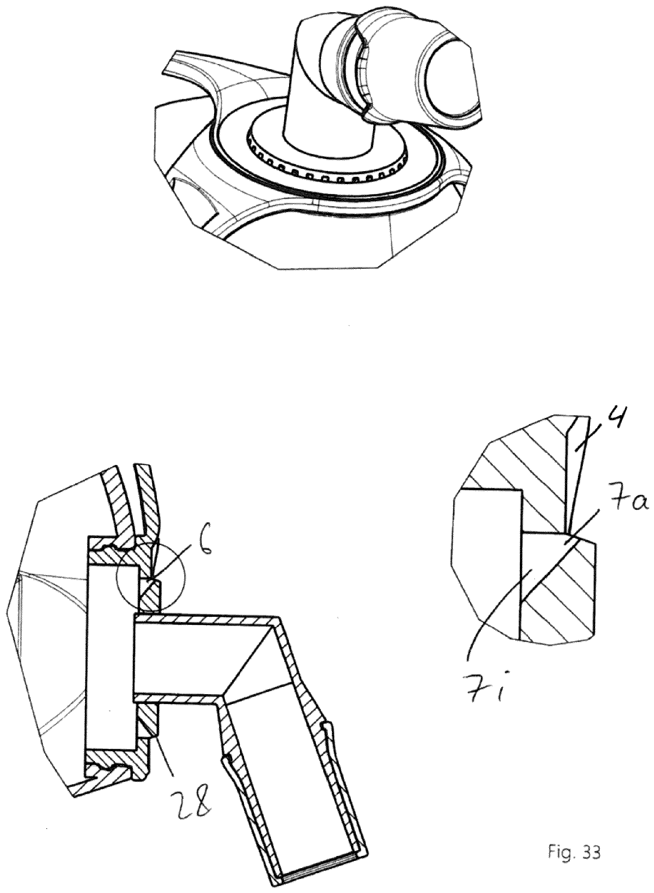

Die Figuren 31 - 34 zeigen die Atemmaske (1) mit einer Maskeneinheit (2) und mit wenigstens einer Ausströmeinrichtung (3) zum Abführen von Ausatemgas aus dem Inneren der Atemmaske (1), wobei die Maskeneinheit (2) wenigstens einen Maskenkörper (4) und wenigstens eine mit dem Maskenkörper (4) verbundene Anschlusseinheit (5) zum Anschließen einer Atemgaszuleitung (101) umfasst und wobei die Ausströmeinrichtung (3) eine Mehrzahl von separaten Strömungskanälen (6) aufweist, welche in dem Maskenkörper (4) benachbart zu der Anschlusseinheit (5) angeordnet sind, wobei die Ausströmeinrichtung (3) zumindest neun separate Strömungskanäle (6) aufweist welche jeweils aus zumindest zwei Teil-Strömungskanälen (7, 7a, 7i) bestehen, wobei die Strömungskanäle (6) voneinander beabstandet und zumindest abschnittsweise kreisförmig um die Anschlusseinheit (5) herum angeordnet sind.

Die Ausströmeinrichtung (3) kann einstückig als Teil des Maskenkörpers (4) oder der Anschlusseinheit ausgebildet sein. Die Ausströmeinrichtung (3) kann auch als separates Bauteil 3 gefertigt sein, welches jedoch in den Maskenkörper oder die Anschlusseinheit integriert ist. Die Ausströmeinrichtung (3) wäre dann austauschbar. Für eine Maske könnten so Ausströmeinrichtungen (3) mit unterschiedlichen Kennlinien verwendet werden.

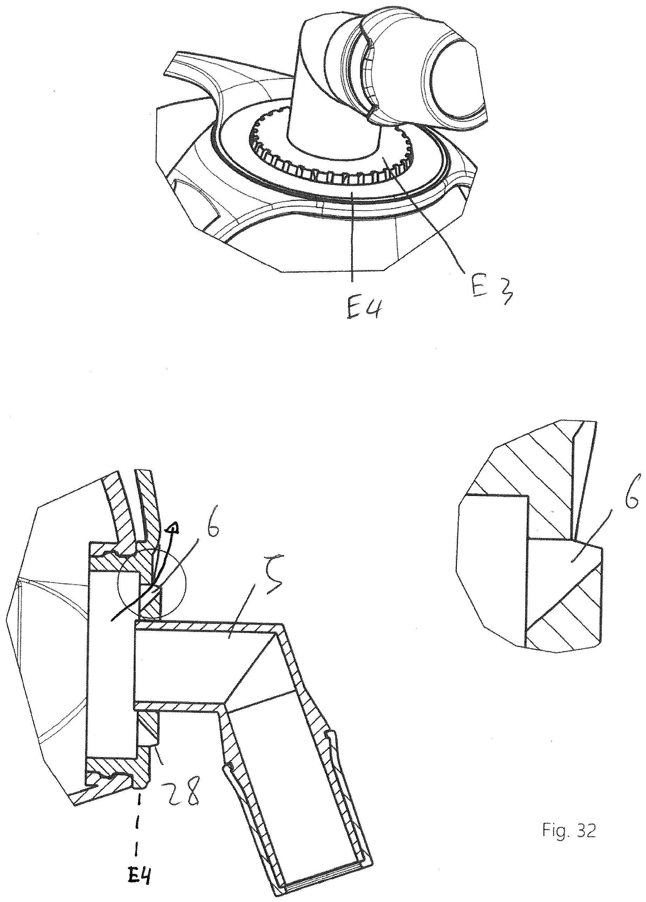

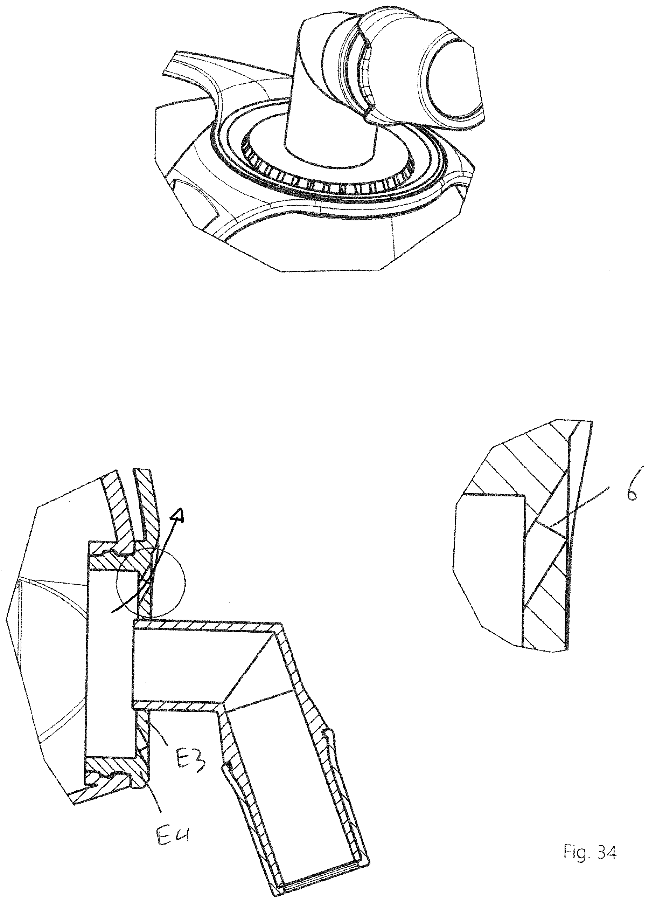

Die Ausströmeinrichtung (3) bildet eine Ebene E3 und der Maskenkörper oder die Anschlusseinheit eine Ebene E4 aus. Die Ebenen E3 und E4 können voneinander beabstandet sein (Fig. 31 und Fig.32) oder können zusammenfallen (Fig. 34). Die Ebenen E3 und E4 können zueinander geneigt verlaufen oder parallel zueinander. Wenn die Ebenen E3 und E4 voneinander beabstandet sind, sind sie materialmäßig durch einen Absatz (28) miteinander verbunden. In dem Absatz 28 können die Strömungskanäle (6) angeordnet sein. Der Absatz kann beliebige Neigungen aufweisen und die Ebenen E3 und E4, beispielsweise mit einer Krümmung oder einer Geraden, verbinden. Durch die Neigung des Absatzes wird auch die Strömungsrichtung bestimmt.

Benachbart zu den Strömungskanälen (6) oder dem Teil-Strömungskanal (7a) weist die Oberfläche des Maskenkörpers (4) eine Strömungsleitfläche 4 auf, die beispielsweise so geneigt ist, dass die abströmenden Atemgase optimal vom Patienten abgeleitet werden. In Figur 32 und 34 sind die abströmenden Atemgase durch einen Pfeil veranschaulicht.

### Bezugszeichenliste:

- 1: Atemmaske
- 2: Maskeneinheit
- 3: Ausströmeinrichtung
- 4: Maskenkörper
- 5: Anschlusseinheit
- 6: Strömungskanal
- 7, 7a, 7i: Teil-Strömungskanal
- 8: Anordnung
- 9: Gießwerkzeug
- 11: Stirnstütze
- 13: Gussteil
- 15: Winkelstück
- 16: Engstellenabschnitt
- 19: Vorsprung
- 21: Maskenwulst
- 26: Einlassbereich
- 28: Absatz
- 36: Auslassbereich
- 100: Beatmungsgerät
- 101: Atemgaszuleitung
- 160: Engstelle
- 200: Gesicht des Patienten
- 210: Innenbereich der Atemmaske
- 260: Einlassöffnung
- 310: Umgebungsluft
- 360: Auslassöffnung

## Patentansprüche

1. Atemmaske (1) mit einer Maskeneinheit (2) und mit wenigstens einer Ausströmeinrichtung (3) zum Abführen von Ausatemgas aus dem Inneren der Atemmaske (1), wobei die Maskeneinheit (2) wenigstens einen Maskenkörper (4) und wenigstens eine mit dem Maskenkörper (4) verbundenen Maskenwulst (21) und eine mit dem Maskenkörper (4) verbundene Anschlusseinheit (5) zum Anschließen einer Atemgaszuleitung (101) umfasst und wobei die Ausströmeinrichtung (3) eine Mehrzahl von separaten Strömungskanälen (6) aufweist, wobei die Ausströmeinrichtung (3) mehrere separate Strömungskanäle (6) aufweist welche jeweils aus zumindest zwei Teil-Strömungskanälen (7, 7a, 7i) bestehen, wobei die Strömungskanäle (6) voneinander beabstandet und zumindest abschnittsweise kreisförmig oder halbkreisförmig angeordnet sind, wobei die Ausströmeinrichtung (3) als separates Bauteil gefertigt ist, welches in den Maskenkörper integriert ist, wobei die Ausströmeinrichtung (3) durch wenigstens ein Gussteil (13) bereitgestellt wird, wobei das Gussteil (13) durch Einpressen mit dem Maskenkörper (4) verbunden ist, wobei die Ausströmeinrichtung (3) an einer Schnittstelle zwischen dem Maskenkörper (4) und der Anschlusseinheit (5) angeordnet ist, **dadurch gekennzeichnet, dass** wenigstens einer der Strömungskanäle (6) wenigstens einen Engstellenabschnitt (16) aufweist und wobei sich der Engstellenabschnitt (16) über wenigstens eine Strecke erstreckt, welche wenigstens ein Achtel der Gesamtlänge des Strömungskanals (6) beträgt und wobei wenigstens einer der Strömungskanäle (6) in Strömungsrichtung vor dem Engstellenabschnitt (16) wenigstens einen sich trichterartig verengenden Einlassbereich (26) und in Strömungsrichtung nach dem Engstellenabschnitt (16) wenigstens einen sich trichterartig aufweitenden Auslassbereich (36) aufweist.

2. Atemmaske (1) nach Anspruch 1 **dadurch gekennzeichnet, dass** die Ausströmeinrichtung (3) eine Ebene E3 definiert und der Maskenkörper oder die Anschlusseinheit eine Ebene E4 definieren, wobei die Ebenen E3 und E4 voneinander beabstandet sind oder zusammenfallen.

3. Atemmaske (1) nach Anspruch 2 **dadurch gekennzeichnet, dass** die Ebenen E3 und E4 voneinander beabstandet sind und materialmäßig durch einen Absatz (28) miteinander verbunden sind, wobei in dem Absatz (28) die Strömungskanäle (6) angeordnet sind.

4. Atemmaske (1) nach zumindest einem der vorhergehenden Ansprüche 2 oder 3 **dadurch gekennzeichnet, dass** die Ebenen E3 und E4 zueinander geneigt verlaufen oder parallel zueinander verlaufen.

5. Atemmaske (1) nach zumindest einem der vorhergehenden Ansprüche **dadurch gekennzeichnet, dass** die Strömungskanäle (6) in dem Maskenkörper (4) benachbart zu der Anschlusseinheit (5) angeordnet sind, **wobei** die Ausströmeinrichtung (3) zumindest neun separate Strömungskanäle (6) aufweist welche jeweils aus zumindest zwei Teil-Strömungskanälen (7, 7a, 7i) bestehen, wobei die Strömungskanäle (6) voneinander beabstandet und zumindest abschnittsweise kreisförmig um die Anschlusseinheit (5) herum angeordnet sind.

6. Atemmaske (1) nach zumindest einem der vorhergehenden Ansprüche **dadurch gekennzeichnet, dass** die Atemmaske (1) in einem Betriebszustand mit dem Maskenwulst (21) abdichtend an dem Gesicht eines Patienten (200) anliegt und dabei einen Überdruck im Inneren der Atemmaske, auf der, dem Patienten zugewandten Seite, im Wesentlichen aufrechthält, wobei die Ausströmeinrichtung (3) mittels der Strömungskanäle (6) unter Überdruck stehendes Ausatemgas aus dem Inneren der Atemmaske (1) über den inneren Teil-Strömungskanal (7i) und den nachfolgenden den äußeren Teil-Strömungskanal (7a) an das Äußere der Atemmaske und in die Umgebungsluft abführt.

7. Atemmaske (1) nach zumindest einem der vorhergehenden Ansprüche **dadurch gekennzeichnet, dass** die Anschlusseinheit (5) drehbar mit dem Maskenkörper (4) verbunden ist und
die Strömungskanäle (6) voneinander beabstandet und zumindest abschnittsweise kreisförmig um die Anschlusseinheit (5) herum angeordnet sind.

8. Atemmaske (1) nach zumindest einem der vorhergehenden Ansprüche **dadurch gekennzeichnet, dass** die Ausströmeinrichtung eine Ebene (E) senkrecht zur Achse (y) der Anschlusseinheit (5) definiert und die Strömungskanäle (6) jeweils eine Ebene (z) der Ausströmrichtung, beispielsweise definiert durch den Bereich ihres kleinsten Querschnitts oder den Teil-Strömungskanal (7a), wobei die Ebenen (E) und (z) in einem Winkel (a) zwischen 20 und 100°, bevorzugt 45 und 90° zueinander angeordnet sind.

9. Atemmaske (1) nach zumindest einem der vorhergehenden Ansprüche **dadurch gekennzeichnet, dass** wenigstens einer der Strömungskanäle (6) einen veränderlichen Kanalquerschnitt und/oder wenigstens zwei unterschiedliche Kanalquerschnitte aufweist, wobei wenigstens einer der Strömungskanäle (6) wenigstens abschnittsweise konisch und/oder kegelförmig ausgebildet ist.

10. Atemmaske (1) nach zumindest einem der vorhergehenden Ansprüche **dadurch gekennzeichnet, dass** wenigstens einer der Strömungskanäle (6) wenigstens abschnittsweise einen Kanalquerschnitt mit einem viereckigen Querschnittsprofil, insbesondere mit abgerundeten Ecken, aufweist.

11. Atemmaske (1) nach zumindest einem der vorhergehenden Ansprüche **dadurch gekennzeichnet, dass** die Strömungskanäle (6) ringförmig an der Maskeneinheit (2) angeordnet sind und wobei die Strömungskanäle (6) über wenigstens einen Teilabschnitt der ringförmigen Anordnung (8) gleichmäßig verteilt sind und über wenigstens einen anderen Teilabschnitt der ringförmigen Anordnung (8) ausgelassen oder verschlossen sind.

12. Verfahren zur Herstellung einer Atemmaske (1) mit einer Maskeneinheit (2) und mit wenigstens einer Ausströmeinrichtung (3) zum Abführen von Ausatemgas aus dem Inneren der Atemmaske (1), wobei die Maskeneinheit (2) wenigstens einen Maskenkörper (4) und wenigstens eine mit dem Maskenkörper (4) verbundenen Maskenwulst (21) und eine mit dem Maskenkörper (4) verbundene Anschlusseinheit (5) zum Anschließen einer Atemgaszuleitung (101) umfasst und wobei die Ausströmeinrichtung (3) eine Mehrzahl von separaten Strömungskanälen (6) aufweist, wobei die Ausströmeinrichtung (3) mehrere separate Strömungskanäle (6) aufweist welche jeweils aus zumindest zwei Teil-Strömungskanälen (7, 7a, 7i) bestehen, wobei die Strömungskanäle (6) voneinander beabstandet und zumindest abschnittsweise kreisförmig oder halbkreisförmig angeordnet sind wobei die Ausströmeinrichtung (3) als separates Bauteil 3 gefertigt ist, welches in den Maskenkörper integriert ist und wobei die Ausströmeinrichtung (3) durch wenigstens ein Gussteil (13) bereitgestellt wird, wobei das Gussteil (13) durch Einpressen mit dem Maskenkörper (4) verbunden ist, wobei die Ausströmeinrichtung (3) an einer Schnittstelle zwischen dem Maskenkörper (4) und der Anschlusseinheit (5) angeordnet ist, wobei zumindest die Ausströmeinrichtung (3) in einem Gießprozess mit wenigstens zwei sich ergänzenden Gießwerkzeugen (9) mit jeweils wenigstens einem während des Gießens wenigstens teilweise den Strömungskanal (6) hineinragenden Vorsprüngen (19) hergestellt ist, **dadurch gekennzeichnet, dass** wenigstens einer der Strömungskanäle (6) wenigstens einen Engstellenabschnitt (16) aufweist und wobei sich der Engstellenabschnitt (16) über wenigstens eine Strecke erstreckt, welche wenigstens ein Achtel der Gesamtlänge des Strömungskanals (6) beträgt und wobei wenigstens einer der Strömungskanäle (6) in Strömungsrichtung vor dem Engstellenabschnitt (16) wenigstens einen sich trichterartig verengenden Einlassbereich (26) und in Strömungsrichtung nach dem Engstellenabschnitt (16) wenigstens einen sich trichterartig aufweitenden Auslassbereich (36) aufweist.

## Claims

1. A breathing mask (1) comprising a mask unit (2) and comprising at least one outflow apparatus (3) for discharging exhaled respiratory gas from inside the breathing mask (1), wherein the mask unit (2) comprises at least one mask body (4) and at least one molded mask portion (21) that is connected to the mask body (4) and a connection unit (5) that is connected to the mask body (4) for connecting a respiratory gas supply line (101) and wherein the outflow apparatus (3) comprises a plurality of separate flow channels (6), wherein the outflow apparatus (3) comprises a plurality of separate flow channels (6) that each consist of at least two sub-flow channels (7, 7a, 7i), wherein the flow channels (6) are arranged so as to be spaced apart from one another and so as to form a circle or semicircle at least in portions, wherein the outflow apparatus (3) is manufactured as a separate component 3 that is integrated in the mask body, wherein the outflow apparatus (3) is provided by means of at least one cast part (13), wherein the cast part (13) is connected to the mask body (4) by means of press-fitting, wherein the outflow apparatus (3) is arranged at an interface between the mask body (4) and the connection unit (5), **characterized in that** at least one of the flow channels (6) comprises at least one constricted portion (16) and wherein the constricted portion (16) extends at least over a distance that corresponds to at least one eighth of the total length of the flow channel (6) and wherein at least one of the flow channels (6) comprises, in flow direction upstream of the constricted portion (16), at least one inlet region (26) that narrows in the manner of a funnel and, in flow direction downstream of the constricted portion (16), at least one outlet region (36) that widens in the manner of a funnel.

2. The breathing mask (1) according to claim 1, **characterized in that** the outflow apparatus (3) defines a plane E3 and the mask body or the connection unit define a plane E4, wherein the planes E3 and E4 are spaced apart from one another or coincide.

3. The breathing mask (1) according to claim 2, **characterized in that** the planes E3 and E4 are spaced apart from one another and are materially connected to one another by means of a stepped portion (28), wherein the flow channels (6) are arranged in the stepped portion (28).

4. The breathing mask (1) according to claim 2 or 3, **characterized in that** the planes E3 and E4 extend so as to be inclined relative to one another or extend in parallel with one another.

5. The breathing mask (1) according to at least one of the preceding claims, **characterized in that** the flow channels (6) in the mask body (4) are arranged so as to adjoin the connection unit (5), **wherein** the outflow apparatus (3) comprises at least nine separate flow channels (6) that each consist of at least two sub-flow channels (7, 7a, 7i), wherein the flow channels (6) are spaced apart from one another and are arranged so as to form a circle around the connection unit (5) at least in portions.

6. The breathing mask (1) according to at least one of the preceding claims, **characterized in that** the breathing mask (1) adjoins the face of a patient (200) in a sealing manner by means of the molded mask portion (21) in a state of operation and thereby substantially maintains a positive pressure inside the breathing mask on the side facing the patient, wherein the outflow apparatus (3) discharges exhaled respiratory gas that is under positive pressure from inside the breathing mask (1) by means of the flow channels (6) via the inner sub-flow channel (7i) and the subsequent outer sub-flow channel (7a) to the outside of the breathing mask and into the surrounding air.

7. The breathing mask (1) according to at least one of the preceding claims, **characterized in that** the connection unit (5) is rotatably connected to the mask body (4) and the flow channels (6) are spaced apart from one another and are arranged so as to form a circle around the connection unit (5) at least in portions.

8. The breathing mask (1) according to at least one of the preceding claims, **characterized in that** the outflow apparatus defines a plane (E) perpendicular to the axis (y) of the connection unit (5) and the flow channels (6) each define a plane (z) of the outflow direction, for example by means of the region of the smallest cross-section thereof or the sub-flow channel (7a), wherein the planes (E) and (z) are arranged at an angle (a) of between 20 and 100°, preferably 45 to 90°, to one another.

9. The breathing mask (1) according to at least one of the preceding claims, **characterized in that** at least one of the flow channels (6) has a variable channel cross-section and/or at least two different channel cross-sections, wherein at least one of the flow channels (6) is designed to be conical and/or tapered at least in portions.

10. The breathing mask (1) according to at least one of the preceding claims, **characterized in that** at least one of the flow channels (6) has, at least in portions, a channel cross-section with a quadrilateral cross-sectional profile, in particular with rounded edges.

11. The breathing mask (1) according to at least one of the preceding claims, **characterized in that** the flow channels (6) are arranged in a ring shape on the mask unit (2) and wherein the flow channels (6) are distributed evenly over at least a subportion of the annular arrangement (8) and are absent or closed over at least another subportion of the annular arrangement (8).

12. A method for manufacturing a breathing mask (1) comprising a mask unit (2) and comprising at least one outflow apparatus (3) for discharging exhaled respiratory gas from inside the breathing mask (1), wherein the mask unit (2) comprises at least one mask body (4) and at least one molded mask portion (21) that is connected to the mask body (4) and a connection unit (5) that is connected to the mask body (4) for connecting a respiratory gas supply line (101) and wherein the outflow apparatus (3) comprises a plurality of separate flow channels (6), wherein the outflow apparatus (3) comprises a plurality of separate flow channels (6) that each consist of at least two sub-flow channels (7, 7a, 7i), wherein the flow channels (6) are arranged so as to be spaced apart from one another and so as to form a circle or semicircle at least in portions, wherein the outflow apparatus (3) is manufactured as a separate component 3 that is integrated in the mask body and wherein the outflow apparatus (3) is provided by means of at least one cast part (13), wherein the cast part (13) is connected to the mask body (4) by means of press-fitting, wherein the outflow apparatus (3) is arranged at an interface between the mask body (4) and the connection unit (5), wherein at least the outflow apparatus (3) is manufactured in a casting process using at least two complementary casting tools (9) each having at least one of the projections (19) that projects at least in part into the flow channel (6) during casting, **characterized in that** at least one of the flow channels (6) comprises at least one constricted portion (16) and wherein the constricted portion (16) extends at least over a distance that corresponds to at least one eighth of the total length of the flow channel (6) and wherein at least one of the flow channels (6) comprises, in flow direction upstream of the constricted portion (16), at least one inlet region (26) that narrows in the manner of a funnel and, in flow direction downstream of the constricted portion (16), at least one outlet region (36) that widens in the manner of a funnel.

## Revendications

1. Masque respiratoire (1) avec une unité de masque (2) et avec au moins un dispositif d'échappement (3) pour évacuer les gaz expirés de l'intérieur du masque respiratoire (1), dans lequel l'unité de masque (2) comprend au moins une coque de masque (4) et au moins un bourrelet de masque (21) relié à la coque de masque (4) et une unité de raccordement (5) reliée à la coque de masque (4) pour rattacher une conduite amenant le gaz respiratoire (101) et dans lequel le dispositif d'échappement (3) présente de multiples canaux d'écoulement séparés (6), dans lequel le dispositif d'échappement (3) présente plusieurs canaux d'écoulement séparés (6), constitués respectivement d'au moins deux canaux d'écoulement partiels (7, 7a, 7i), dans lequel les canaux d'écoulement (6) sont espacés les uns des autres et disposés au moins par endroits en cercle ou en demi-cercle, dans lequel le dispositif d'échappement (3) est fabriqué comme un composant séparé 3, lequel est intégré à la coque de masque, dans lequel le dispositif d'échappement (3) est fourni au moins par une pièce coulée (13), dans lequel la pièce coulée (13) est reliée à la coque de masque (4) par enfoncement, dans lequel le dispositif d'échappement (3) est disposé à une interface entre la coque de masque (4) et l'unité de raccordement (5), **caractérisé en ce qu'**au moins l'un des canaux d'écoulement (6) présente au moins un tronçon rétréci (16) et dans lequel le tronçon rétréci (16) s'étend sur au moins une distance qui représente au moins un huitième de la longueur totale du canal d'écoulement (6) et dans lequel au moins l'un des canaux d'écoulement (6) présente au moins une zone d'admission (26) se resserrant en entonnoir dans le sens de l'écoulement en amont du tronçon rétréci (16) et au moins une zone d'évacuation (36) s'élargissant en entonnoir dans le sens de l'écoulement en aval du tronçon rétréci (16).

2. Masque respiratoire (1) selon la revendication 1 **caractérisé en ce que** le dispositif d'échappement (3) définit un plan E3 et la coque de masque ou l'unité de raccordement définissent un plan E4, dans lequel les plans E3 et E4 sont espacés l'un de l'autre ou coïncident.

3. Masque respiratoire (1) selon la revendication 2 **caractérisé en ce que** les plans E3 et E4 sont espacés l'un de l'autre et sont reliés matériellement l'un à l'autre par un épaulement (28), dans lequel les canaux d'écoulement (6) sont disposés à l'intérieur de l'épaulement (28).

4. Masque respiratoire (1) selon la revendication 2 ou 3 **caractérisé en ce que** les plans E3 et E4 s'étendent en inclinaison l'un vers l'autre ou parallèlement l'un à l'autre.

5. Masque respiratoire (1) selon au moins l'une des revendications précédentes **caractérisé en ce que** les canaux d'écoulement (6) sont disposés dans la coque de masque (4) à proximité de l'unité de raccordement (5), dans lequel le dispositif d'échappement (3) présente au moins neuf canaux d'écoulement séparés (6) constitués respectivement d'au moins deux canaux d'écoulement partiels (7, 7a, 7i), dans lequel les canaux d'écoulement (6) sont espacés les uns des autres et disposés au moins par endroits en cercle autour de l'unité de raccordement (5).

6. Masque respiratoire (1) selon au moins l'une des revendications précédentes **caractérisé en ce que** le masque respiratoire (1) repose de manière étanche sur le visage d'un patient (200) moyennant le bourrelet de masque (21) dans un état opérationnel et maintient alors essentiellement une surpression à l'intérieur du masque respiratoire du côté tourné vers la patient, dans lequel le dispositif d'échappement (3) évacue les gaz expirés en surpression de l'intérieur du masque respiratoire (1) à l'extérieur du masque respiratoire et dans l'air ambiant au moyen des canaux d'écoulement (6) via le canal d'écoulement partiel intérieur (7i) et le canal d'écoulement partiel extérieur qui suit (7a).

7. Masque respiratoire (1) selon au moins l'une des revendications précédentes **caractérisé en ce que** l'unité de raccordement (5) est reliée de façon pivotante à la coque de masque (4) et les canaux d'écoulement (6) sont espacés les uns des autres et disposés au moins par endroits en cercle autour de l'unité de raccordement (5).

8. Masque respiratoire (1) selon au moins l'une des revendications précédentes **caractérisé en ce que** le dispositif d'échappement définit un plan (E) perpendiculaire à l'axe (y) de l'unité de raccordement (5) et les canaux d'écoulement (6) définissent respectivement un plan (z) du sens d'échappement, par exemple moyennant la zone de leur plus petite section transversale ou le canal d'écoulement partiel (7a), dans lequel les plans (E) et (z) sont disposés à un angle (a) compris entre 20° et 100°, de préférence entre 45° et 90° l'un par rapport à l'autre.

9. Masque respiratoire (1) selon au moins l'une des revendications précédentes **caractérisé en ce qu'**au moins l'un des canaux d'écoulement (6) présente une section transversale variable et/ou au moins deux sections transversales différentes, dans lequel au moins l'un des canaux d'écoulement (6) est conçu au moins par endroits sous une forme conique et/ou de cône.

10. Masque respiratoire (1) selon au moins l'une des revendications précédentes **caractérisé en ce qu'**au moins l'un des canaux d'écoulement (6) présente au moins par endroits une section transversale ayant un profil carré, notamment des angles arrondis.

11. Masque respiratoire (1) selon au moins l'une des revendications précédentes **caractérisé en ce que** les canaux d'écoulement (6) sont disposés en forme d'anneau sur l'unité de masque (2) et dans lequel les canaux d'écoulement (6) sont répartis uniformément sur au moins une partie de la disposition annulaire (8) et sont laissés de côté ou fermés sur au moins une autre partie de la disposition annulaire (8).

12. Procédé de fabrication d'un masque respiratoire (1) avec une unité de masque (2) et avec au moins un dispositif d'échappement (3) pour évacuer les gaz expirés de l'intérieur du masque respiratoire (1), dans lequel l'unité de masque (2) comprend au moins une coque de masque (4) et au moins un bourrelet de masque (21) relié à la coque de masque (4) et une unité de raccordement (5) reliée à la coque de masque (4) pour rattacher une conduite amenant le gaz respiratoire (101) et dans lequel le dispositif d'échappement (3) présente de multiples canaux d'écoulement séparés (6), dans lequel le dispositif d'échappement (3) présente plusieurs canaux d'écoulement séparés (6), constitués respectivement d'au moins deux canaux d'écoulement partiels (7, 7a, 7i), dans lequel les canaux d'écoulement (6) sont espacés les uns des autres et disposés au moins par endroits en cercle ou en demi-cercle, dans lequel le dispositif d'échappement (3) est fabriqué comme un composant séparé 3, lequel est intégré à la coque de masque, dans lequel le dispositif d'échappement (3) est fourni au moins par une pièce coulée (13), dans lequel la pièce coulée (13) est reliée à la coque de masque (4) par enfoncement, dans lequel le dispositif d'échappement (3) est disposé à une interface entre la coque de masque (4) et l'unité de raccordement (5), dans lequel au moins le dispositif d'échappement (3) est fabriqué au cours d'un processus de coulée au moyen d'au moins deux outils de coulée se complétant (9) avec respectivement au moins l'une des saillies (19) dépassant au moins par endroits du canal d'écoulement (6) durant la coulée, **caractérisé en ce qu'**au moins l'un des canaux d'écoulement (6) présente au moins un tronçon rétréci (16) et dans lequel le tronçon rétréci (16) s'étend sur au moins une distance qui représente au moins un huitième de la longueur totale du canal d'écoulement (6) et dans lequel au moins l'un des canaux d'écoulement (6) présente au moins une zone d'admission (26) se resserrant en entonnoir dans le sens de l'écoulement en amont du tronçon rétréci (16) et au moins une zone d'évacuation (36) s'élargissant en entonnoir dans le sens de l'écoulement en aval du tronçon rétréci (16).
